# EUROPÄISCHE PATENTSCHRIFT

(11) **EP 3 014 279 B1**
(45) Veröffentlichungstag und Bekanntmachung des Hinweises auf die Patenterteilung: **12.10.2022**
(21) Anmeldenummer: 14752793.1
(22) Anmeldetag: 25.06.2014
(51) Int. Cl.: G01N 33/68, C07K 14/435, G01N 33/543, G01N 21/64, C07K 14/47

(54) **VERFAHREN ZUR BESTIMMUNG VON PROTEINAGGREGATEN UNTER VERWENDUNG VON OBERFLÄCHEN-FIDA**
METHOD FOR DETERMINING PROTEIN AGGREGATES USING SURFACE-FIDA
PROCÉDÉ DE DÉTECTION D'AGGRÉGATS DE PROTÉINES À L'AIDE DE LA TECHNIQUE SURFACE-FIDA

(30) Priorität: 26.06.2013 DE 102013106713
(43) Veröffentlichungstag der Anmeldung: 04.05.2016
(73) Patentinhaber: Forschungszentrum Jülich GmbH, 52425 Jülich (DE)
(72) Erfinder: WILLBOLD, Dieter, 52428 Jülich (DE); BIRKMANN, Eva, 40589 Düsseldorf (DE); KRAVCHENKO, Kateryna, 45279 Essen (DE); FUNKE, Aileen, 96242 Sonnefeld (DE); BANNACH, Oliver, 40595 Düsseldorf (DE); HÜBINGER, Steffen, 40217 Düsseldorf (DE); KORTH, Carsten, 40219 Düsseldorf (DE); BADER, Verian, 45657 Recklinghausen (DE)
(74) Vertreter: Fitzner, Uwe
(86) Internationale Anmeldenummer: PCT/EP2014/063418
(87) Internationale Veröffentlichungsnummer: WO 2014/207049

(56) Entgegenhaltungen:
- WO-A2-2007/126473
- WO-A2-2007/126473
- WO-A2-2008/070229
- WO-A2-2008/070229
- WO-A2-2008/070229
- US-A1- 2008 220 449
- US-A1- 2008 220 449
- US-A1- 2009 042 211
- S. HÜBINGER ET AL: "Detection of [alpha]-Synuclein Aggregates by Fluorescence Microscopy", REJUVENATION RESEARCH, Bd. 15, Nr. 2, 1. April 2012 (2012-04-01), Seiten 213-216, XP055522916, US ISSN: 1549-1684, DOI: 10.1089/rej.2011.1288
- TOKUDA T ET AL: "Detection of elevated levels of alpha-synuclein oligomers in CSF from patients with Parkinson disease", NEUROLOGY, LIPPINCOTT WILLIAMS & WILKINS, PHILADELPHIA, US, Bd. 75, Nr. 20, 1. November 2010 (2010-11-01), Seiten 1766-1772, XP002635208, ISSN: 0028-3878, DOI: 10.1212/WNL.0B013E3181FD613B [gefunden am 2010-10-20]
- VOLLES ET AL: "Relationships between the Sequence of @a-Synuclein and its Membrane Affinity, Fibrillization Propensity, and Yeast Toxicity", JOURNAL OF MOLECULAR BIO, ACADEMIC PRESS, UNITED KINGDOM, Bd. 366, Nr. 5, 9. Februar 2007 (2007-02-09), Seiten 1510-1522, XP005874847, ISSN: 0022-2836, DOI: 10.1016/J.JMB.2006.12.044
- MICHAEL R. SIERKS ET AL: "CSF levels of oligomeric alpha-synuclein and beta-amyloid as biomarkers for neurodegenerative disease", INTEGRATIVE BIOLOGY, Bd. 3, Nr. 12, 10. November 2011 (2011-11-10), Seiten 1188-1196, XP055563484, DOI: 10.1039/c1ib00018g
- FUNKE ET AL: "Single particle detection of Abeta aggregates associated with Alzheimer's disease", BIOCHEMICAL AND BIOPHYSICAL RESEARCH COMMUNICATIONS, ACADEMIC PRESS INC. ORLANDO, FL, US, Bd. 364, Nr. 4, 24. Oktober 2007 (2007-10-24), Seiten 902-907, XP022344209, ISSN: 0006-291X, DOI: 10.1016/J.BBRC.2007.10.085
- SUSANNE AILEEN FUNKE ET AL: "An Ultrasensitive Assay for Diagnosis of Alzheimer's Disease", REJUVENATION RESEARCH, Bd. 11, Nr. 2, 1. April 2008 (2008-04-01), Seiten 315-318, XP055060114, ISSN: 1549-1684, DOI: 10.1089/rej.2008.0670
- SUSANNE AILEEN FUNKE ET AL: "Single-Particle Detection System for Ab Aggregates: Adaptation of Surface-Fluorescence Intensity Distribution Analysis to Laser Scanning Microscopy", REJUVENATION RESEARCH, Bd. 13, Nr. 2-3, 4. Dezember 2009 (2009-12-04), Seiten 206-209, XP055060118, DOI: 10.1089=rej.2009.0925
- WANG-DIETRICHET: "The amyloid-beta oligomer count in cerebrospinal fluid is a biomarker for Alzheimer's disease", JOURNAL OF ALZHEIMER'S DISEASE, Bd. 34, 11. Januar 2013 (2013-01-11), Seiten 985-994, XP055060188, in der Anmeldung erwähnt
- Pilar Infiesta ET AL: "Development of an assay to measure tau oligomer levels in AD", 42nd Annual Meeting of the Society-for-Neuroscience; New Orleans, LA, USA, 17. Oktober 2012 (2012-10-17), XP055147762, Gefunden im Internet: URL:http://www.oligomerix.com/frames/tech/ Oligomerix%20Tau%20Biomarker%20Poster%20fo r%20SfN%202012%20-%20FINAL.pdf [gefunden am 2014-10-20]
- S. HÜBINGER ET AL: "Detection of [alpha]-Synuclein Aggregates by Fluorescence Microscopy", REJUVENATION RESEARCH, vol. 15, no. 2, 1 April 2012 (2012-04-01), pages 213-216, XP055522916, US ISSN: 1549-1684, DOI: 10.1089/rej.2011.1288
- TOKUDA T ET AL: "Detection of elevated levels of alpha-synuclein oligomers in CSF from patients with Parkinson disease", NEUROLOGY, LIPPINCOTT WILLIAMS & WILKINS, PHILADELPHIA, US, vol. 75, no. 20, 1 November 2010 (2010-11-01), pages 1766-1772, XP002635208, ISSN: 0028-3878, DOI: 10.1212/WNL.0B013E3181FD613B [retrieved on 2010-10-20]
- VOLLES ET AL: "Relationships between the Sequence of @a-Synuclein and its Membrane Affinity, Fibrillization Propensity, and Yeast Toxicity", JOURNAL OF MOLECULAR BIO, ACADEMIC PRESS, UNITED KINGDOM, vol. 366, no. 5, 9 February 2007 (2007-02-09), pages 1510-1522, XP005874847, ISSN: 0022-2836, DOI: 10.1016/J.JMB.2006.12.044
- MICHAEL R. SIERKS ET AL: "CSF levels of oligomeric alpha-synuclein and beta-amyloid as biomarkers for neurodegenerative disease", INTEGRATIVE BIOLOGY, vol. 3, no. 12, 10 November 2011 (2011-11-10), pages 1188-1196, XP055563484, DOI: 10.1039/c1ib00018g
- Pedersen Jeppe T. ET AL: "Analysis of Protein Aggregation in Neurodegenerative Disease", Analytical Chemistry, vol. 85, no. 9, 7 May 2013 (2013-05-07), pages 4215-4227, XP055801225, US ISSN: 0003-2700, DOI: 10.1021/ac400023c Retrieved from the Internet: URL:https://pubs.acs.org/doi/pdf/10.1021/a c400023c>
- Takashi Kasai ET AL: "Utilization of a multiple antigenic peptide as a calibration standard in the BAN50 single antibody sandwich ELISA for A[beta] oligomers", Biochemical and Biophysical Research Communications, vol. 422, no. 3, 1 June 2012 (2012-06-01), pages 375-380, XP055756071, Amsterdam NL ISSN: 0006-291X, DOI: 10.1016/j.bbrc.2012.04.146
- EL-AGNAF OMAR M A ET AL: "Detection of oligomeric forms of alpha-synuclein protein in human plasma as a potential biomarker for Parkinson's disease", FASEB JOURNAL,, vol. 20, no. 3, 1 March 2006 (2006-03-01), pages 419-425, XP009109458, DOI: 10.1096/FJ.03-1449COM

### SEQUENCE LISTING

<110> Forschungszentrum Juelich
<120> Verfahren zur Ermittlung von Indikatoren zur Bestimmung von Krankheiten
<130> FZJ 1303 PCT
<160> 15
<170> PatentIn version 3.5
<210> 1
   <211> 122
   <212> PRT
   <213> Homo sapiens
<400> 1
   Met Lys Leu Leu Thr Gly Leu Val Phe Cys Ser Leu Val Leu Gly Val
   1               5                   10                  15
   Ser Ser Arg Ser Phe Phe Ser Phe Leu Gly Glu Ala Phe Asp Gly Ala
               20                  25                  30
   Arg Asp Met Trp Arg Ala Tyr Ser Asp Met Arg Glu Ala Asn Tyr Ile
           35                  40                  45
   Gly Ser Asp Lys Tyr Phe His Ala Arg Gly Asn Tyr Asp Ala Ala Lys
       50                  55                  60
   Arg Gly Pro Gly Gly Val Trp Ala Ala Glu Ala Ile Ser Asp Ala Arg
   65                  70                  75                  80
   Glu Asn Ile Gln Arg Phe Phe Gly His Gly Ala Glu Asp Ser Leu Ala
                   85                  90                  95
   Asp Gln Ala Ala Asn Glu Trp Gly Arg Ser Gly Lys Asp Pro Asn His
               100                 105                 110
   Phe Arg Pro Ala Gly Leu Pro Glu Lys Tyr
           115                 120
<210> 2
   <211> 213
   <212> PRT
   <213> Homo sapiens
<400> 2
   Met Arg Pro Gly Thr Gly Gln Gly Gly Leu Glu Ala Pro Gly Glu Pro
   1               5                   10                  15
   Gly Pro Asn Leu Arg Gln Arg Trp Pro Leu Leu Leu Leu Gly Leu Ala
               20                  25                  30
   Val Val Thr His Gly Leu Leu Arg Pro Thr Ala Ala Ser Gln Ser Arg
           35                  40                  45
   Ala Leu Gly Pro Gly Ala Pro Gly Gly Ser Ser Arg Ser Ser Leu Arg
       50                  55                  60
   Ser Arg Trp Gly Arg Phe Leu Leu Gln Arg Gly Ser Trp Thr Gly Pro
   65                  70                  75                  80
   Arg Cys Trp Pro Arg Gly Phe Gln Ser Lys His Asn Ser Val Thr His
                   85                  90                  95
   Val Phe Gly Ser Gly Thr Gln Leu Thr Val Leu Ser Gln Pro Lys Ala
               100                 105                 110
   Thr Pro Ser Val Thr Leu Phe Pro Pro Ser Ser Glu Glu Leu Gln Ala
           115                 120                 125
   Asn Lys Ala Thr Leu Val Cys Leu Met Asn Asp Phe Tyr Pro Gly Ile
       130                 135                 140
   Leu Thr Val Thr Trp Lys Ala Asp Gly Thr Pro Ile Thr Gln Gly Val
   145                 150                 155                 160
   Glu Met Thr Thr Pro Ser Lys Gln Ser Asn Asn Lys Tyr Ala Ala Ser
                   165                 170                 175
   Ser Tyr Leu Ser Leu Thr Pro Glu Gln Trp Arg Ser Arg Arg Ser Tyr
               180                 185                 190
   Ser Cys Gln Val Met His Glu Gly Ser Thr Val Glu Lys Thr Val Ala
           195                 200                 205
   Pro Ala Glu Cys Ser
       210
<210> 3
   <211> 267
   <212> PRT
   <213> Homo sapiens
<400> 3
<210> 4
   <211> 100
   <212> PRT
   <213> Homo sapiens
<400> 4
   Met Lys Leu Leu Ala Ala Thr Val Leu Leu Leu Thr Ile Cys Ser Leu
   1               5                   10                  15
   Glu Gly Ala Leu Val Arg Arg Gln Ala Lys Glu Pro Cys Val Glu Ser
               20                  25                  30
   Leu Val Ser Gln Tyr Phe Gln Thr Val Thr Asp Tyr Gly Lys Asp Leu
           35                  40                  45
   Met Glu Lys Val Lys Ser Pro Glu Leu Gln Ala Glu Ala Lys Ser Tyr
       50                  55                  60
   Phe Glu Lys Ser Lys Glu Gln Leu Thr Pro Leu Ile Lys Lys Ala Gly
   65                  70                  75                  80
   Thr Glu Leu Val Asn Phe Leu Ser Tyr Phe Val Glu Leu Gly Thr Gln
                   85                  90                  95
   Pro Ala Thr Gln
               100
<210> 5
   <211> 147
   <212> PRT
   <213> Homo sapiens
<400> 5
<210> 6
   <211> 854
   <212> PRT
   <213> Homo sapiens
<400> 6
   Met Pro Gly Gly Gly Pro Gln Gly Ala Pro Ala Ala Ala Gly Gly Gly
   1 5 10 15
   Gly Val Ser His Arg Ala Gly Ser Arg Asp Cys Leu Pro Pro Ala Ala
   20 25 30
   Cys Phe Arg Arg Arg Arg Leu Ala Arg Arg Pro Gly Tyr Met Arg Ser
   35 40 45
   Ser Thr Gly Pro Gly Ile Gly Phe Leu Ser Pro Ala Val Gly Thr Leu
   50 55 60
   Phe Arg Phe Pro Gly Gly Val Ser Gly Glu Glu Ser His His Ser Glu
   65 70 75 80
   Ser Arg Ala Arg Gln Cys Gly Leu Asp Ser Arg Gly Leu Leu Val Arg
   85 90 95
   Ser Pro Val Ser Lys Ser Ala Ala Ala Pro Thr Val Thr Ser Val Arg
   100 105 110
   Gly Thr Ser Ala His Phe Gly Ile Gln Leu Arg Gly Gly Thr Arg Leu
   115 120 125
   Pro Asp Arg Leu Ser Trp Pro Cys Gly Pro Gly Ser Ala Gly Trp Gln
   130 135 140
   Gln Glu Phe Ala Ala Met Asp Ser Ser Glu Thr Leu Asp Ala Ser Trp
   145                 150                 155                 160
   Glu Ala Ala Cys Ser Asp Gly Ala Arg Arg Val Arg Ala Ala Gly Ser
                   165                 170                 175
   Leu Pro Ser Ala Glu Leu Ser Ser Asn Ser Cys Ser Pro Gly Cys Gly
               180                 185                 190
   Pro Glu Val Pro Pro Thr Pro Pro Gly Ser His Ser Ala Phe Thr Ser
           195                 200                 205
   Ser Phe Ser Phe Ile Arg Leu Ser Leu Gly Ser Ala Gly Glu Arg Gly
       210                 215                 220
   Glu Ala Glu Gly Cys Pro Pro Ser Arg Glu Ala Glu Ser His Cys Gln
   225                 230                 235                 240
   Ser Pro Gln Glu Met Gly Ala Lys Ala Ala Ser Leu Asp Gly Pro His
                   245                 250                 255
   Glu Asp Pro Arg Cys Leu Ser Arg Pro Phe Ser Leu Leu Ala Thr Arg
               260                 265                 270
   Val Ser Ala Asp Leu Ala Gln Ala Ala Arg Asn Ser Ser Arg Pro Glu
           275                 280                 285
   Arg Asp Met His Ser Leu Pro Asp Met Asp Pro Gly Ser Ser Ser Ser
       290                 295                 300
   Leu Asp Pro Ser Leu Ala Gly Cys Gly Gly Asp Gly Ser Ser Gly Ser
   305                 310                 315                 320
   Gly Asp Ala His Ser Trp Asp Thr Leu Leu Arg Lys Trp Glu Pro Val
                   325                 330                 335
   Leu Arg Asp Cys Leu Leu Arg Asn Arg Arg Gln Met Glu Val Ile Ser
               340                 345                 350
   Leu Arg Leu Lys Leu Gln Lys Leu Gln Glu Asp Ala Val Glu Asn Asp
           355                 360                 365
   Asp Tyr Asp Lys Ala Glu Thr Leu Gln Gln Arg Leu Glu Asp Leu Glu
       370                 375                 380
   Gln Glu Lys Ile Ser Leu His Phe Gln Leu Pro Ser Arg Gln Pro Ala
   385                 390                 395                 400
   Leu Ser Ser Phe Leu Gly His Leu Ala Ala Gln Val Gln Ala Ala Leu
                   405                 410                 415
   Arg Arg Gly Ala Thr Gln Gln Ala Ser Gly Asp Asp Thr His Thr Pro
               420                 425                 430
   Leu Arg Met Glu Pro Arg Leu Leu Glu Pro Thr Ala Gln Asp Ser Leu
           435                 440                 445
   His Val Ser Ile Thr Arg Arg Asp Trp Leu Leu Gln Glu Lys Gln Gln
       450                 455                 460
   Leu Gln Lys Glu Ile Glu Ala Leu Gln Ala Arg Met Phe Val Leu Glu
   465                 470                 475                 480
   Ala Lys Asp Gln Gln Leu Arg Arg Glu Ile Glu Glu Gln Glu Gln Gln
                   485                 490                 495
   Leu Gln Trp Gln Gly Cys Asp Leu Thr Pro Leu Val Gly Gln Leu Ser
               500                 505                 510
   Leu Gly Gln Leu Gln Glu Val Ser Lys Ala Leu Gln Asp Thr Leu Ala
           515                 520                 525
   Ser Ala Gly Gln Ile Pro Phe His Ala Glu Pro Pro Glu Thr Ile Arg
       530                 535                 540
   Ser Leu Gln Glu Arg Ile Lys Ser Leu Asn Leu Ser Leu Lys Glu Ile
   545                 550                 555                 560
   Thr Thr Lys Val Cys Met Ser Glu Lys Phe Cys Ser Thr Leu Arg Lys
                   565                 570                 575
   Lys Val Asn Asp Ile Glu Thr Gln Leu Pro Ala Leu Leu Glu Ala Lys
               580                 585                 590
   Met His Ala Ile Ser Gly Asn His Phe Trp Thr Ala Lys Asp Leu Thr
           595                 600                 605
   Glu Glu Ile Arg Ser Leu Thr Ser Glu Arg Glu Gly Leu Glu Gly Leu
       610                 615                 620
   Leu Ser Lys Leu Leu Val Leu Ser Ser Arg Asn Val Lys Lys Leu Gly
   625                 630                 635                 640
   Ser Val Lys Glu Asp Tyr Asn Arg Leu Arg Arg Glu Val Glu His Gln
                   645                 650                 655
   Glu Thr Ala Tyr Glu Thr Ser Val Lys Glu Asn Thr Met Lys Tyr Met
               660                 665                 670
   Glu Thr Leu Lys Asn Lys Leu Cys Ser Cys Lys Cys Pro Leu Leu Gly
           675                 680                 685
   Lys Val Trp Glu Ala Asp Leu Glu Ala Cys Arg Leu Leu Ile Gln Ser
       690                 695                 700
   Leu Gln Leu Gln Glu Ala Arg Gly Ser Leu Ser Val Glu Asp Glu Arg
   705                 710                 715                 720
   Gln Met Asp Asp Leu Glu Gly Ala Ala Pro Pro Ile Pro Pro Arg Leu
                   725                 730                 735
   His Ser Glu Asp Lys Arg Lys Thr Pro Leu Lys Val Leu Glu Glu Trp
               740                 745                 750
   Lys Thr His Leu Ile Pro Ser Leu His Cys Ala Gly Gly Glu Gln Lys
           755                 760                 765
   Glu Glu Ser Tyr Ile Leu Ser Ala Glu Leu Gly Glu Lys Cys Glu Asp
       770                 775                 780
   Ile Gly Lys Lys Leu Leu Tyr Leu Glu Asp Gln Leu His Thr Ala Ile
   785                 790                 795                 800
   His Ser His Asp Glu Asp Leu Ile Gln Ser Leu Arg Arg Glu Leu Gln
                   805                 810                 815
   Met Val Lys Glu Thr Leu Gln Ala Met Ile Leu Gln Leu Gln Pro Ala
               820                 825                 830
   Lys Glu Ala Gly Glu Arg Glu Ala Ala Ala Ser Cys Met Thr Ala Gly
           835                 840                 845
   Val His Glu Ala Gln Ala
       850
<210> 7
   <211> 526
   <212> PRT
   <213> Homo sapiens
<400> 7
<210> 8
   <211> 89
   <212> PRT
   <213> Homo sapiens
<400> 8
   Met Gly Ile Leu Lys Leu Gln Val Phe Leu Ile Val Leu Ser Val Ala
   1 5 10 15
   Leu Asn His Leu Lys Ala Thr Pro Ile Glu Ser His Gln Val Glu Lys
   20 25 30
   Arg Lys Cys Asn Thr Ala Thr Cys Ala Thr Gln Arg Leu Ala Asn Phe
   35 40 45
   Leu Val His Ser Ser Asn Asn Phe Gly Ala Ile Leu Ser Ser Thr Asn
   50 55 60
   Val Gly Ser Asn Thr Tyr Gly Lys Arg Asn Ala Val Glu Val Leu Lys
   65 70 75 80
   Arg Glu Pro Leu Asn Tyr Leu Pro Leu
   85
<210> 9
   <211> 154
   <212> PRT
   <213> Homo sapiens
<400> 9
<210> 10
   <211> 140
   <212> PRT
   <213> Homo sapiens
<400> 10
<210> 11
   <211> 758
   <212> PRT
   <213> Homo sapiens
<400> 11
   Met Ala Glu Pro Arg Gln Glu Phe Glu Val Met Glu Asp His Ala Gly
   1               5                   10                  15
   Thr Tyr Gly Leu Gly Asp Arg Lys Asp Gln Gly Gly Tyr Thr Met His
               20                  25                  30
   Gln Asp Gln Glu Gly Asp Thr Asp Ala Gly Leu Lys Glu Ser Pro Leu
           35                  40                  45
   Gln Thr Pro Thr Glu Asp Gly Ser Glu Glu Pro Gly Ser Glu Thr Ser
       50                  55                  60
   Asp Ala Lys Ser Thr Pro Thr Ala Glu Asp Val Thr Ala Pro Leu Val
   65                  70                  75                  80
   Asp Glu Gly Ala Pro Gly Lys Gln Ala Ala Ala Gln Pro His Thr Glu
                   85                  90                  95
   Ile Pro Glu Gly Thr Thr Ala Glu Glu Ala Gly Ile Gly Asp Thr Pro
               100                 105                 110
   Ser Leu Glu Asp Glu Ala Ala Gly His Val Thr Gln Glu Pro Glu Ser
           115                 120                 125
   Gly Lys Val Val Gln Glu Gly Phe Leu Arg Glu Pro Gly Pro Pro Gly
       130                 135                 140
   Leu Ser His Gln Leu Met Ser Gly Met Pro Gly Ala Pro Leu Leu Pro
   145                 150                 155                 160
   Glu Gly Pro Arg Glu Ala Thr Arg Gln Pro Ser Gly Thr Gly Pro Glu
                   165                 170                 175
   Asp Thr Glu Gly Gly Arg His Ala Pro Glu Leu Leu Lys His Gln Leu
               180                 185                 190
   Leu Gly Asp Leu His Gln Glu Gly Pro Pro Leu Lys Gly Ala Gly Gly
           195                 200                 205
   Lys Glu Arg Pro Gly Ser Lys Glu Glu Val Asp Glu Asp Arg Asp Val
       210                 215                 220
   Asp Glu Ser Ser Pro Gln Asp Ser Pro Pro Ser Lys Ala Ser Pro Ala
   225                 230                 235                 240
   Gln Asp Gly Arg Pro Pro Gln Thr Ala Ala Arg Glu Ala Thr Ser Ile
                   245                 250                 255
   Pro Gly Phe Pro Ala Glu Gly Ala Ile Pro Leu Pro Val Asp Phe Leu
               260                 265                 270
   Ser Lys Val Ser Thr Glu Ile Pro Ala Ser Glu Pro Asp Gly Pro Ser
           275                 280                 285
   Val Gly Arg Ala Lys Gly Gln Asp Ala Pro Leu Glu Phe Thr Phe His
       290                 295                 300
   Val Glu Ile Thr Pro Asn Val Gln Lys Glu Gln Ala His Ser Glu Glu
   305                 310                 315                 320
   His Leu Gly Arg Ala Ala Phe Pro Gly Ala Pro Gly Glu Gly Pro Glu
                   325                 330                 335
   Ala Arg Gly Pro Ser Leu Gly Glu Asp Thr Lys Glu Ala Asp Leu Pro
               340                 345                 350
   Glu Pro Ser Glu Lys Gln Pro Ala Ala Ala Pro Arg Gly Lys Pro Val
           355                 360                 365
   Ser Arg Val Pro Gln Leu Lys Ala Arg Met Val Ser Lys Ser Lys Asp
       370                 375                 380
   Gly Thr Gly Ser Asp Asp Lys Lys Ala Lys Thr Ser Thr Arg Ser Ser
   385                 390                 395                 400
   Ala Lys Thr Leu Lys Asn Arg Pro Cys Leu Ser Pro Lys His Pro Thr
                   405                 410                 415
   Pro Gly Ser Ser Asp Pro Leu Ile Gln Pro Ser Ser Pro Ala Val Cys
               420                 425                 430
   Pro Glu Pro Pro Ser Ser Pro Lys Tyr Val Ser Ser Val Thr Ser Arg
           435                 440                 445
   Thr Gly Ser Ser Gly Ala Lys Glu Met Lys Leu Lys Gly Ala Asp Gly
       450                 455                 460
   Lys Thr Lys Ile Ala Thr Pro Arg Gly Ala Ala Pro Pro Gly Gln Lys
   465                 470                 475                 480
   Gly Gln Ala Asn Ala Thr Arg Ile Pro Ala Lys Thr Pro Pro Ala Pro
                   485                 490                 495
   Lys Thr Pro Pro Ser Ser Gly Glu Pro Pro Lys Ser Gly Asp Arg Ser
               500                 505                 510
   Gly Tyr Ser Ser Pro Gly Ser Pro Gly Thr Pro Gly Ser Arg Ser Arg
           515                 520                 525
   Thr Pro Ser Leu Pro Thr Pro Pro Thr Arg Glu Pro Lys Lys Val Ala
       530                 535                 540
   Val Val Arg Thr Pro Pro Lys Ser Pro Ser Ser Ala Lys Ser Arg Leu
   545                 550                 555                 560
   Gln Thr Ala Pro Val Pro Met Pro Asp Leu Lys Asn Val Lys Ser Lys
                   565                 570                 575
   Ile Gly Ser Thr Glu Asn Leu Lys His Gln Pro Gly Gly Gly Lys Val
               580                 585                 590
   Gln Ile Ile Asn Lys Lys Leu Asp Leu Ser Asn Val Gln Ser Lys Cys
           595                 600                 605
   Gly Ser Lys Asp Asn Ile Lys His Val Pro Gly Gly Gly Ser Val Gln
       610                 615                 620
   Ile Val Tyr Lys Pro Val Asp Leu Ser Lys Val Thr Ser Lys Cys Gly
   625                 630                 635                 640
   Ser Leu Gly Asn Ile His His Lys Pro Gly Gly Gly Gln Val Glu Val
                   645                 650                 655
   Lys Ser Glu Lys Leu Asp Phe Lys Asp Arg Val Gln Ser Lys Ile Gly
               660                 665                 670
   Ser Leu Asp Asn Ile Thr His Val Pro Gly Gly Gly Asn Lys Lys Ile
           675                 680                 685
   Glu Thr His Lys Leu Thr Phe Arg Glu Asn Ala Lys Ala Lys Thr Asp
       690                 695                 700
   His Gly Ala Glu Ile Val Tyr Lys Ser Pro Val Val Ser Gly Asp Thr
   705                 710                 715                 720
   Ser Pro Arg His Leu Ser Asn Val Ser Ser Thr Gly Ser Ile Asp Met
                   725                 730                 735
   Val Asp Ser Pro Gln Leu Ala Thr Leu Ala Asp Glu Val Ser Ala Ser
   740 745 750
   Leu Ala Lys Gln Gly Leu
   755
<210> 12
   <211> 414
   <212> PRT
   <213> Homo sapiens
<400> 12
<210> 13
   <211> 3142
   <212> PRT
   <213> Homo sapiens
<400> 13
   Met Ala Thr Leu Glu Lys Leu Met Lys Ala Phe Glu Ser Leu Lys Ser
   1               5                   10                  15
   Phe Gln Gln Gln Gln Gln Gln Gln Gln Gln Gln Gln Gln Gln Gln Gln
               20                  25                  30
   Gln Gln Gln Gln Gln Gln Pro Pro Pro Pro Pro Pro Pro Pro Pro Pro
           35                  40                  45
   Pro Gln Leu Pro Gln Pro Pro Pro Gln Ala Gln Pro Leu Leu Pro Gln
       50                  55                  60
   Pro Gln Pro Pro Pro Pro Pro Pro Pro Pro Pro Pro Gly Pro Ala Val
   65                  70                  75                  80
   Ala Glu Glu Pro Leu His Arg Pro Lys Lys Glu Leu Ser Ala Thr Lys
                   85                  90                  95
   Lys Asp Arg Val Asn His Cys Leu Thr Ile Cys Glu Asn Ile Val Ala
               100                 105                 110
   Gln Ser Val Arg Asn Ser Pro Glu Phe Gln Lys Leu Leu Gly Ile Ala
           115                 120                 125
   Met Glu Leu Phe Leu Leu Cys Ser Asp Asp Ala Glu Ser Asp Val Arg
       130                 135                 140
   Met Val Ala Asp Glu Cys Leu Asn Lys Val Ile Lys Ala Leu Met Asp
   145                 150                 155                 160
   Ser Asn Leu Pro Arg Leu Gln Leu Glu Leu Tyr Lys Glu Ile Lys Lys
                   165                 170                 175
   Asn Gly Ala Pro Arg Ser Leu Arg Ala Ala Leu Trp Arg Phe Ala Glu
               180                 185                 190
   Leu Ala His Leu Val Arg Pro Gln Lys Cys Arg Pro Tyr Leu Val Asn
           195                 200                 205
   Leu Leu Pro Cys Leu Thr Arg Thr Ser Lys Arg Pro Glu Glu Ser Val
       210                 215                 220
   Gln Glu Thr Leu Ala Ala Ala Val Pro Lys Ile Met Ala Ser Phe Gly
   225                 230                 235                 240
   Asn Phe Ala Asn Asp Asn Glu Ile Lys Val Leu Leu Lys Ala Phe Ile
                   245                 250                 255
   Ala Asn Leu Lys Ser Ser Ser Pro Thr Ile Arg Arg Thr Ala Ala Gly
               260                 265                 270
   Ser Ala Val Ser Ile Cys Gln His Ser Arg Arg Thr Gln Tyr Phe Tyr
           275                 280                 285
   Ser Trp Leu Leu Asn Val Leu Leu Gly Leu Leu Val Pro Val Glu Asp
       290                 295                 300
   Glu His Ser Thr Leu Leu Ile Leu Gly Val Leu Leu Thr Leu Arg Tyr
   305                 310                 315                 320
   Leu Val Pro Leu Leu Gln Gln Gln Val Lys Asp Thr Ser Leu Lys Gly
                   325                 330                 335
   Ser Phe Gly Val Thr Arg Lys Glu Met Glu Val Ser Pro Ser Ala Glu
               340                 345                 350
   Gln Leu Val Gln Val Tyr Glu Leu Thr Leu His His Thr Gln His Gln
           355                 360                 365
   Asp His Asn Val Val Thr Gly Ala Leu Glu Leu Leu Gln Gln Leu Phe
       370                 375                 380
   Arg Thr Pro Pro Pro Glu Leu Leu Gln Thr Leu Thr Ala Val Gly Gly
   385                 390                 395                 400
   Ile Gly Gln Leu Thr Ala Ala Lys Glu Glu Ser Gly Gly Arg Ser Arg
                   405                 410                 415
   Ser Gly Ser Ile Val Glu Leu Ile Ala Gly Gly Gly Ser Ser Cys Ser
               420                 425                 430
   Pro Val Leu Ser Arg Lys Gln Lys Gly Lys Val Leu Leu Gly Glu Glu
           435                 440                 445
   Glu Ala Leu Glu Asp Asp Ser Glu Ser Arg Ser Asp Val Ser Ser Ser
       450                 455                 460
   Ala Leu Thr Ala Ser Val Lys Asp Glu Ile Ser Gly Glu Leu Ala Ala
   465                 470                 475                 480
   Ser Ser Gly Val Ser Thr Pro Gly Ser Ala Gly His Asp Ile Ile Thr
                   485                 490                 495
   Glu Gln Pro Arg Ser Gln His Thr Leu Gln Ala Asp Ser Val Asp Leu
   500 505 510
   Ala Ser Cys Asp Leu Thr Ser Ser Ala Thr Asp Gly Asp Glu Glu Asp
   515 520 525
   Ile Leu Ser His Ser Ser Ser Gln Val Ser Ala Val Pro Ser Asp Pro
   530 535 540
   Ala Met Asp Leu Asn Asp Gly Thr Gln Ala Ser Ser Pro Ile Ser Asp
   545 550 555 560
   Ser Ser Gln Thr Thr Thr Glu Gly Pro Asp Ser Ala Val Thr Pro Ser
   565 570 575
   Asp Ser Ser Glu Ile Val Leu Asp Gly Thr Asp Asn Gln Tyr Leu Gly
   580 585 590
   Leu Gln Ile Gly Gln Pro Gln Asp Glu Asp Glu Glu Ala Thr Gly Ile
   595 600 605
   Leu Pro Asp Glu Ala Ser Glu Ala Phe Arg Asn Ser Ser Met Ala Leu
   610 615 620
   Gln Gln Ala His Leu Leu Lys Asn Met Ser His Cys Arg Gln Pro Ser
   625 630 635 640
   Asp Ser Ser Val Asp Lys Phe Val Leu Arg Asp Glu Ala Thr Glu Pro
   645 650 655
   Gly Asp Gln Glu Asn Lys Pro Cys Arg Ile Lys Gly Asp Ile Gly Gln
   660 665 670
   Ser Thr Asp Asp Asp Ser Ala Pro Leu Val His Cys Val Arg Leu Leu
   675 680 685
   Ser Ala Ser Phe Leu Leu Thr Gly Gly Lys Asn Val Leu Val Pro Asp
   690 695 700
   Arg Asp Val Arg Val Ser Val Lys Ala Leu Ala Leu Ser Cys Val Gly
   705 710 715 720
   Ala Ala Val Ala Leu His Pro Glu Ser Phe Phe Ser Lys Leu Tyr Lys
   725 730 735
   Val Pro Leu Asp Thr Thr Glu Tyr Pro Glu Glu Gln Tyr Val Ser Asp
   740 745 750
   Ile Leu Asn Tyr Ile Asp His Gly Asp Pro Gln Val Arg Gly Ala Thr
   755 760 765
   Ala Ile Leu Cys Gly Thr Leu Ile Cys Ser Ile Leu Ser Arg Ser Arg
   770 775 780
   Phe His Val Gly Asp Trp Met Gly Thr Ile Arg Thr Leu Thr Gly Asn
   785 790 795 800
   Thr Phe Ser Leu Ala Asp Cys Ile Pro Leu Leu Arg Lys Thr Leu Lys
   805 810 815
   Asp Glu Ser Ser Val Thr Cys Lys Leu Ala Cys Thr Ala Val Arg Asn
   820 825 830
   Cys Val Met Ser Leu Cys Ser Ser Ser Tyr Ser Glu Leu Gly Leu Gln
   835 840 845
   Leu Ile Ile Asp Val Leu Thr Leu Arg Asn Ser Ser Tyr Trp Leu Val
   850 855 860
   Arg Thr Glu Leu Leu Glu Thr Leu Ala Glu Ile Asp Phe Arg Leu Val
   865 870 875 880
   Ser Phe Leu Glu Ala Lys Ala Glu Asn Leu His Arg Gly Ala His His
   885 890 895
   Tyr Thr Gly Leu Leu Lys Leu Gln Glu Arg Val Leu Asn Asn Val Val
   900 905 910
   Ile His Leu Leu Gly Asp Glu Asp Pro Arg Val Arg His Val Ala Ala
   915 920 925
   Ala Ser Leu Ile Arg Leu Val Pro Lys Leu Phe Tyr Lys Cys Asp Gln
   930 935 940
   Gly Gln Ala Asp Pro Val Val Ala Val Ala Arg Asp Gln Ser Ser Val
   945 950 955 960
   Tyr Leu Lys Leu Leu Met His Glu Thr Gln Pro Pro Ser His Phe Ser
   965 970 975
   Val Ser Thr Ile Thr Arg Ile Tyr Arg Gly Tyr Asn Leu Leu Pro Ser
   980 985 990
   Ile Thr Asp Val Thr Met Glu Asn Asn Leu Ser Arg Val Ile Ala Ala
   995 1000 1005
   Val Ser  His Glu Leu Ile Thr  Ser Thr Thr Arg Ala  Leu Thr Phe
       1010                 1015                 1020
   Gly Cys  Cys Glu Ala Leu Cys  Leu Leu Ser Thr Ala  Phe Pro Val
       1025                 1030                 1035
   Cys Ile  Trp Ser Leu Gly Trp  His Cys Gly Val Pro  Pro Leu Ser
       1040                 1045                 1050
   Ala Ser  Asp Glu Ser Arg Lys  Ser Cys Thr Val Gly  Met Ala Thr
       1055                 1060                 1065
   Met Ile  Leu Thr Leu Leu Ser  Ser Ala Trp Phe Pro  Leu Asp Leu
       1070                 1075                 1080
   Ser Ala  His Gln Asp Ala Leu  Ile Leu Ala Gly Asn  Leu Leu Ala
       1085                 1090                 1095
   Ala Ser  Ala Pro Lys Ser Leu  Arg Ser Ser Trp Ala  Ser Glu Glu
       1100                 1105                 1110
   Glu Ala  Asn Pro Ala Ala Thr  Lys Gln Glu Glu Val  Trp Pro Ala
       1115                 1120                 1125
   Leu Gly  Asp Arg Ala Leu Val  Pro Met Val Glu Gln  Leu Phe Ser
       1130                 1135                 1140
   His Leu  Leu Lys Val Ile Asn  Ile Cys Ala His Val  Leu Asp Asp
       1145                 1150                 1155
   Val Ala  Pro Gly Pro Ala Ile  Lys Ala Ala Leu Pro  Ser Leu Thr
       1160                 1165                 1170
   Asn Pro  Pro Ser Leu Ser Pro  Ile Arg Arg Lys Gly  Lys Glu Lys
       1175                 1180                 1185
   Glu Pro  Gly Glu Gln Ala Ser  Val Pro Leu Ser Pro  Lys Lys Gly
       1190                 1195                 1200
   Ser Glu  Ala Ser Ala Ala Ser  Arg Gln Ser Asp Thr  Ser Gly Pro
       1205                 1210                 1215
   Val Thr  Thr Ser Lys Ser Ser  Ser Leu Gly Ser Phe  Tyr His Leu
       1220                 1225                 1230
   Pro Ser  Tyr Leu Lys Leu His  Asp Val Leu Lys Ala  Thr His Ala
       1235                 1240                 1245
   Asn Tyr  Lys Val Thr Leu Asp  Leu Gln Asn Ser Thr  Glu Lys Phe
       1250                 1255                 1260
   Gly Gly  Phe Leu Arg Ser Ala  Leu Asp Val Leu Ser  Gln Ile Leu
       1265                 1270                 1275
   Glu Leu  Ala Thr Leu Gln Asp  Ile Gly Lys Cys Val  Glu Glu Ile
       1280                 1285                 1290
   Leu Gly  Tyr Leu Lys Ser Cys  Phe Ser Arg Glu Pro  Met Met Ala
       1295                 1300                 1305
   Thr Val  Cys Val Gln Gln Leu  Leu Lys Thr Leu Phe  Gly Thr Asn
       1310                 1315                 1320
   Leu Ala  Ser Gln Phe Asp Gly  Leu Ser Ser Asn Pro  Ser Lys Ser
       1325                 1330                 1335
   Gln Gly  Arg Ala Gln Arg Leu  Gly Ser Ser Ser Val  Arg Pro Gly
       1340                 1345                 1350
   Leu Tyr  His Tyr Cys Phe Met  Ala Pro Tyr Thr His  Phe Thr Gln
       1355                 1360                 1365
   Ala Leu  Ala Asp Ala Ser Leu  Arg Asn Met Val Gln  Ala Glu Gln
       1370                 1375                 1380
   Glu Asn  Asp Thr Ser Gly Trp  Phe Asp Val Leu Gln  Lys Val Ser
       1385                 1390                 1395
   Thr Gln  Leu Lys Thr Asn Leu  Thr Ser Val Thr Lys  Asn Arg Ala
       1400                 1405                 1410
   Asp Lys  Asn Ala Ile His Asn  His Ile Arg Leu Phe  Glu Pro Leu
       1415                 1420                 1425
   Val Ile  Lys Ala Leu Lys Gln  Tyr Thr Thr Thr Thr  Cys Val Gln
       1430                 1435                 1440
   Leu Gln  Lys Gln Val Leu Asp  Leu Leu Ala Gln Leu  Val Gln Leu
       1445                 1450                 1455
   Arg Val  Asn Tyr Cys Leu Leu  Asp Ser Asp Gln Val  Phe Ile Gly
       1460                 1465                 1470
   Phe Val  Leu Lys Gln Phe Glu  Tyr Ile Glu Val Gly  Gln Phe Arg
   1475 1480 1485
   Glu Ser Glu Ala Ile Ile Pro Asn Ile Phe Phe Phe Leu Val Leu
   1490 1495 1500
   Leu Ser Tyr Glu Arg Tyr His Ser Lys Gln Ile Ile Gly Ile Pro
   1505 1510 1515
   Lys Ile Ile Gln Leu Cys Asp Gly Ile Met Ala Ser Gly Arg Lys
   1520 1525 1530
   Ala Val Thr His Ala Ile Pro Ala Leu Gln Pro Ile Val His Asp
   1535 1540 1545
   Leu Phe Val Leu Arg Gly Thr Asn Lys Ala Asp Ala Gly Lys Glu
   1550 1555 1560
   Leu Glu Thr Gln Lys Glu Val Val Val Ser Met Leu Leu Arg Leu
   1565 1570 1575
   Ile Gln Tyr His Gln Val Leu Glu Met Phe Ile Leu Val Leu Gln
   1580 1585 1590
   Gln Cys His Lys Glu Asn Glu Asp Lys Trp Lys Arg Leu Ser Arg
   1595 1600 1605
   Gln Ile Ala Asp Ile Ile Leu Pro Met Leu Ala Lys Gln Gln Met
   1610 1615 1620
   His Ile Asp Ser His Glu Ala Leu Gly Val Leu Asn Thr Leu Phe
   1625 1630 1635
   Glu Ile Leu Ala Pro Ser Ser Leu Arg Pro Val Asp Met Leu Leu
   1640 1645 1650
   Arg Ser Met Phe Val Thr Pro Asn Thr Met Ala Ser Val Ser Thr
   1655 1660 1665
   Val Gln Leu Trp Ile Ser Gly Ile Leu Ala Ile Leu Arg Val Leu
   1670 1675 1680
   Ile Ser Gln Ser Thr Glu Asp Ile Val Leu Ser Arg Ile Gln Glu
   1685 1690 1695
   Leu Ser Phe Ser Pro Tyr Leu Ile Ser Cys Thr Val Ile Asn Arg
   1700 1705 1710
   Leu Arg  Asp Gly Asp Ser Thr  Ser Thr Leu Glu Glu  His Ser Glu
       1715                 1720                 1725
   Gly Lys  Gln Ile Lys Asn Leu  Pro Glu Glu Thr Phe  Ser Arg Phe
       1730                 1735                 1740
   Leu Leu  Gln Leu Val Gly Ile  Leu Leu Glu Asp Ile  Val Thr Lys
       1745                 1750                 1755
   Gln Leu  Lys Val Glu Met Ser  Glu Gln Gln His Thr  Phe Tyr Cys
       1760                 1765                 1770
   Gln Glu  Leu Gly Thr Leu Leu  Met Cys Leu Ile His  Ile Phe Lys
       1775                 1780                 1785
   Ser Gly  Met Phe Arg Arg Ile  Thr Ala Ala Ala Thr  Arg Leu Phe
       1790                 1795                 1800
   Arg Ser  Asp Gly Cys Gly Gly  Ser Phe Tyr Thr Leu  Asp Ser Leu
       1805                 1810                 1815
   Asn Leu  Arg Ala Arg Ser Met  Ile Thr Thr His Pro  Ala Leu Val
       1820                 1825                 1830
   Leu Leu  Trp Cys Gln Ile Leu  Leu Leu Val Asn His  Thr Asp Tyr
       1835                 1840                 1845
   Arg Trp  Trp Ala Glu Val Gln  Gln Thr Pro Lys Arg  His Ser Leu
       1850                 1855                 1860
   Ser Ser  Thr Lys Leu Leu Ser  Pro Gln Met Ser Gly  Glu Glu Glu
       1865                 1870                 1875
   Asp Ser  Asp Leu Ala Ala Lys  Leu Gly Met Cys Asn  Arg Glu Ile
       1880                 1885                 1890
   Val Arg  Arg Gly Ala Leu Ile  Leu Phe Cys Asp Tyr  Val Cys Gln
       1895                 1900                 1905
   Asn Leu  His Asp Ser Glu His  Leu Thr Trp Leu Ile  Val Asn His
       1910                 1915                 1920
   Ile Gln  Asp Leu Ile Ser Leu  Ser His Glu Pro Pro  Val Gln Asp
       1925                 1930                 1935
   Phe Ile  Ser Ala Val His Arg  Asn Ser Ala Ala Ser  Gly Leu Phe
       1940                 1945                 1950
   Ile Gln Ala Ile Gln Ser Arg Cys Glu Asn Leu Ser Thr Pro Thr
   1955 1960 1965
   Met Leu Lys Lys Thr Leu Gln Cys Leu Glu Gly Ile His Leu Ser
   1970 1975 1980
   Gln Ser Gly Ala Val Leu Thr Leu Tyr Val Asp Arg Leu Leu Cys
   1985 1990 1995
   Thr Pro Phe Arg Val Leu Ala Arg Met Val Asp Ile Leu Ala Cys
   2000 2005 2010
   Arg Arg Val Glu Met Leu Leu Ala Ala Asn Leu Gln Ser Ser Met
   2015 2020 2025
   Ala Gln Leu Pro Met Glu Glu Leu Asn Arg Ile Gln Glu Tyr Leu
   2030 2035 2040
   Gln Ser Ser Gly Leu Ala Gln Arg His Gln Arg Leu Tyr Ser Leu
   2045 2050 2055
   Leu Asp Arg Phe Arg Leu Ser Thr Met Gln Asp Ser Leu Ser Pro
   2060 2065 2070
   Ser Pro Pro Val Ser Ser His Pro Leu Asp Gly Asp Gly His Val
   2075 2080 2085
   Ser Leu Glu Thr Val Ser Pro Asp Lys Asp Trp Tyr Val His Leu
   2090 2095 2100
   Val Lys Ser Gln Cys Trp Thr Arg Ser Asp Ser Ala Leu Leu Glu
   2105 2110 2115
   Gly Ala Glu Leu Val Asn Arg Ile Pro Ala Glu Asp Met Asn Ala
   2120 2125 2130
   Phe Met Met Asn Ser Glu Phe Asn Leu Ser Leu Leu Ala Pro Cys
   2135 2140 2145
   Leu Ser Leu Gly Met Ser Glu Ile Ser Gly Gly Gln Lys Ser Ala
   2150 2155 2160
   Leu Phe Glu Ala Ala Arg Glu Val Thr Leu Ala Arg Val Ser Gly
   2165 2170 2175
   Thr Val Gln Gln Leu Pro Ala Val His His Val Phe Gln Pro Glu
   2180 2185 2190
   Leu Pro  Ala Glu Pro Ala Ala  Tyr Trp Ser Lys Leu  Asn Asp Leu
       2195                 2200                 2205
   Phe Gly  Asp Ala Ala Leu Tyr  Gln Ser Leu Pro Thr  Leu Ala Arg
       2210                 2215                 2220
   Ala Leu  Ala Gln Tyr Leu Val  Val Val Ser Lys Leu  Pro Ser His
       2225                 2230                 2235
   Leu His  Leu Pro Pro Glu Lys  Glu Lys Asp Ile Val  Lys Phe Val
       2240                 2245                 2250
   Val Ala  Thr Leu Glu Ala Leu  Ser Trp His Leu Ile  His Glu Gln
       2255                 2260                 2265
   Ile Pro  Leu Ser Leu Asp Leu  Gln Ala Gly Leu Asp  Cys Cys Cys
       2270                 2275                 2280
   Leu Ala  Leu Gln Leu Pro Gly  Leu Trp Ser Val Val  Ser Ser Thr
       2285                 2290                 2295
   Glu Phe  Val Thr His Ala Cys  Ser Leu Ile Tyr Cys  Val His Phe
       2300                 2305                 2310
   Ile Leu  Glu Ala Val Ala Val  Gln Pro Gly Glu Gln  Leu Leu Ser
       2315                 2320                 2325
   Pro Glu  Arg Arg Thr Asn Thr  Pro Lys Ala Ile Ser  Glu Glu Glu
       2330                 2335                 2340
   Glu Glu  Val Asp Pro Asn Thr  Gln Asn Pro Lys Tyr  Ile Thr Ala
       2345                 2350                 2355
   Ala Cys  Glu Met Val Ala Glu  Met Val Glu Ser Leu  Gln Ser Val
       2360                 2365                 2370
   Leu Ala  Leu Gly His Lys Arg  Asn Ser Gly Val Pro  Ala Phe Leu
       2375                 2380                 2385
   Thr Pro  Leu Leu Arg Asn Ile  Ile Ile Ser Leu Ala  Arg Leu Pro
       2390                 2395                 2400
   Leu Val  Asn Ser Tyr Thr Arg  Val Pro Pro Leu Val  Trp Lys Leu
       2405                 2410                 2415
   Gly Trp  Ser Pro Lys Pro Gly  Gly Asp Phe Gly Thr  Ala Phe Pro
   2420 2425 2430
   Glu Ile Pro Val Glu Phe Leu Gln Glu Lys Glu Val Phe Lys Glu
   2435 2440 2445
   Phe Ile Tyr Arg Ile Asn Thr Leu Gly Trp Thr Ser Arg Thr Gln
   2450 2455 2460
   Phe Glu Glu Thr Trp Ala Thr Leu Leu Gly Val Leu Val Thr Gln
   2465 2470 2475
   Pro Leu Val Met Glu Gln Glu Glu Ser Pro Pro Glu Glu Asp Thr
   2480 2485 2490
   Glu Arg Thr Gln Ile Asn Val Leu Ala Val Gln Ala Ile Thr Ser
   2495 2500 2505
   Leu Val Leu Ser Ala Met Thr Val Pro Val Ala Gly Asn Pro Ala
   2510 2515 2520
   Val Ser Cys Leu Glu Gln Gln Pro Arg Asn Lys Pro Leu Lys Ala
   2525 2530 2535
   Leu Asp Thr Arg Phe Gly Arg Lys Leu Ser Ile Ile Arg Gly Ile
   2540 2545 2550
   Val Glu Gln Glu Ile Gln Ala Met Val Ser Lys Arg Glu Asn Ile
   2555 2560 2565
   Ala Thr His His Leu Tyr Gln Ala Trp Asp Pro Val Pro Ser Leu
   2570 2575 2580
   Ser Pro Ala Thr Thr Gly Ala Leu Ile Ser His Glu Lys Leu Leu
   2585 2590 2595
   Leu Gln Ile Asn Pro Glu Arg Glu Leu Gly Ser Met Ser Tyr Lys
   2600 2605 2610
   Leu Gly Gln Val Ser Ile His Ser Val Trp Leu Gly Asn Ser Ile
   2615 2620 2625
   Thr Pro Leu Arg Glu Glu Glu Trp Asp Glu Glu Glu Glu Glu Glu
   2630 2635 2640
   Ala Asp Ala Pro Ala Pro Ser Ser Pro Pro Thr Ser Pro Val Asn
   2645 2650 2655
   Ser Arg Lys His Arg Ala Gly Val Asp Ile His Ser Cys Ser Gln
   2660 2665 2670
   Phe Leu Leu Glu Leu Tyr Ser Arg Trp Ile Leu Pro Ser Ser Ser
   2675 2680 2685
   Ala Arg Arg Thr Pro Ala Ile Leu Ile Ser Glu Val Val Arg Ser
   2690 2695 2700
   Leu Leu Val Val Ser Asp Leu Phe Thr Glu Arg Asn Gln Phe Glu
   2705 2710 2715
   Leu Met Tyr Val Thr Leu Thr Glu Leu Arg Arg Val His Pro Ser
   2720 2725 2730
   Glu Asp Glu Ile Leu Ala Gln Tyr Leu Val Pro Ala Thr Cys Lys
   2735 2740 2745
   Ala Ala Ala Val Leu Gly Met Asp Lys Ala Val Ala Glu Pro Val
   2750 2755 2760
   Ser Arg Leu Leu Glu Ser Thr Leu Arg Ser Ser His Leu Pro Ser
   2765 2770 2775
   Arg Val Gly Ala Leu His Gly Val Leu Tyr Val Leu Glu Cys Asp
   2780 2785 2790
   Leu Leu Asp Asp Thr Ala Lys Gln Leu Ile Pro Val Ile Ser Asp
   2795 2800 2805
   Tyr Leu Leu Ser Asn Leu Lys Gly Ile Ala His Cys Val Asn Ile
   2810 2815 2820
   His Ser Gln Gln His Val Leu Val Met Cys Ala Thr Ala Phe Tyr
   2825 2830 2835
   Leu Ile Glu Asn Tyr Pro Leu Asp Val Gly Pro Glu Phe Ser Ala
   2840 2845 2850
   Ser Ile Ile Gln Met Cys Gly Val Met Leu Ser Gly Ser Glu Glu
   2855 2860 2865
   Ser Thr Pro Ser Ile Ile Tyr His Cys Ala Leu Arg Gly Leu Glu
   2870 2875 2880
   Arg Leu Leu Leu Ser Glu Gln Leu Ser Arg Leu Asp Ala Glu Ser
   2885 2890 2895
   Leu Val Lys Leu Ser Val Asp Arg Val Asn Val His Ser Pro His
       2900                2905                2910
   Arg Ala Met Ala Ala Leu Gly Leu Met Leu Thr Cys Met Tyr Thr
       2915                2920                2925
   Gly Lys Glu Lys Val Ser Pro Gly Arg Thr Ser Asp Pro Asn Pro
       2930                2935                2940
   Ala Ala Pro Asp Ser Glu Ser Val Ile Val Ala Met Glu Arg Val
       2945                2950                2955
   Ser Val Leu Phe Asp Arg Ile Arg Lys Gly Phe Pro Cys Glu Ala
       2960                2965                2970
   Arg Val Val Ala Arg Ile Leu Pro Gln Phe Leu Asp Asp Phe Phe
       2975                2980                2985
   Pro Pro Gln Asp Ile Met Asn Lys Val Ile Gly Glu Phe Leu Ser
       2990                2995                3000
   Asn Gln Gln Pro Tyr Pro Gln Phe Met Ala Thr Val Val Tyr Lys
       3005                3010                3015
   Val Phe Gln Thr Leu His Ser Thr Gly Gln Ser Ser Met Val Arg
       3020                3025                3030
   Asp Trp Val Met Leu Ser Leu Ser Asn Phe Thr Gln Arg Ala Pro
       3035                3040                3045
   Val Ala Met Ala Thr Trp Ser Leu Ser Cys Phe Phe Val Ser Ala
       3050                3055                3060
   Ser Thr Ser Pro Trp Val Ala Ala Ile Leu Pro His Val Ile Ser
       3065                3070                3075
   Arg Met Gly Lys Leu Glu Gln Val Asp Val Asn Leu Phe Cys Leu
       3080                3085                3090
   Val Ala Thr Asp Phe Tyr Arg His Gln Ile Glu Glu Glu Leu Asp
       3095                3100                3105
   Arg Arg Ala Phe Gln Ser Val Leu Glu Val Val Ala Ala Pro Gly
       3110                3115                3120
   Ser Pro Tyr His Arg Leu Leu Thr Cys Leu Arg Asn Val His Lys
       3125                3130                3135
   Val Thr Thr Cys
   3140
<210> 14
   <211> 148
   <212> PRT
   <213> Homo sapiens
<400> 14
<210> 15
   <211> 43
   <212> PRT
   <213> Homo sapiens
<400> 15
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
               20                  25                  30
   Gly Leu Met Val Gly Gly Val Val Ile Ala Thr
           35                  40

## Beschreibung

Die Erfindung betrifft ein Verfahren zur qualitativen und/oder quantitativen Bestimmung von Krankheitsindikatoren, in denen Aggregate fehlgefalteter Proteine eine Rolle spielen, die Verwendung eines Standards zur selektiven Quantifizierung und/oder Charakterisierung der Krankheitsindikatoren in diesem Verfahren, ein Verfahren zur Bestimmung von Krankheiten die Aggregate fehlgefalteter Proteine aufweisen, sowie ein Kit zur Verwendung in einem solchen Verfahren.

Pathologische Aggregate aus körpereigenen Proteinen, wie z.B. Oligomere oder Fibrillen, treten in vielen neurodegenerativen Erkrankungen auf.

Amyloidosen sind Krankheiten, die durch extrazelluläre Ablagerungen von unlöslichen, fehlgefalteten Proteinen charakterisiert sind. Diese Aggregate liegen in der Regel als Fasern, sog. Beta-Fibrillen vor. Der Begriff Amyloid (=stärkeähnlich) leitet sich von den spektralphotometrischen Gemeinsamkeiten von Kongorot-gefärbten Fibrillen und Stärke ab. Die Ätiologie der Amyloidosen kann sowohl hereditär als auch spontan sein. Auch treten einige Amyloidosen als Sekundärerkrankungen auf.

Bei der Alzheimer-Krankheit (AD, Alzheimersche Demenz, lateinisch = Morbus Alzheimer) treten A-Beta-Aggregate auf. AD gehört neben Morbus Parkinson zu einer heterogenen Gruppe klinischer Zustände, deren gemeinsames Kriterium in vielen Fällen (aber nicht ausschließlich) extrazelluläre, systemische oder lokale Ablagerungen eines jeweils spezifischen Proteins ist, meist in beta-Faltblattreichen Konformationen.

Die Amyloid-Beta-Peptid-Ablagerungen (oder Peptid-Fibrillen) stellen allerdings lediglich das Endstadium eines Prozesses dar. Pathologische Hauptmerkmale der AD sind die Bildung von senilen oder amyloiden Plaques, bestehend aus dem A-Beta-Peptid, und zusätzlichen neurofibrillären Ablagerungen aus dem Tau-Protein. Das Vorläufer-Protein des A-Beta-Peptids, APP, ist in der Zellwand von Neuronen lokalisiert. Durch proteolytischen Abbau und nachträgliche Modifikation entstehen daraus A-Beta-Fragmente unterschiedlicher Länge und Art, wie z.B. A-Beta 1-40, A-Beta 1-42 oder pGluA-Beta 3-42. Monomere A-Beta-Peptide entstehen während des gesamten Lebens auch im gesunden Organismus.

Gemäß der Amyloid-Kaskaden-Hypothese aus den 1990er Jahren sind die A-Beta-Ablagerungen in Form von Plaques die Auslöser der Krankheitssymptome. In den letzten Jahren weisen jedoch unterschiedliche Studien darauf hin, dass besonders die kleinen, frei diffundierenden A-Beta-Oligomere die größte Toxizität unter allen A-Beta-Spezies besitzen und für die Entstehung und den Fortschritt der AD verantwortlich sind. Somit sind Aggregate des A-Beta-Peptids unmittelbar mit der AD-Pathogenese verknüpft und möglicherweise als Biomarker für AD zu verwenden (Wang-Dietrich et al., J. Alzheimer's Disease 34, 2013, 985-994). Eine Unterstützung der Diagnose durch ein weiteres Routineverfahren wäre allerdings hilfreich, insbesondere zur Abgrenzung von anderen Krankheiten basierend auf körpereigenen fehlgefalteten Proteinen.

Protein-Aggregate werden ferner in WO 2005/018424 A1, WO 2008/070229 A2, DE 10 2006 010 647 A1, Bannach et al. (Plosone, May 2012, Vol. 7, issue 5, e36620), WO 2010/003227 A1, DE 10 2011 057 021 A1 und WO 2013/092952 A2 (veröffentlich 27.06.2013) erwähnt.

Die AA-Amyloidose ist eine recht seltene Folgeerkrankung bei chronisch-entzündlicher Darmerkrankung, wie Colitis ulcerosa und Morbus Crohn. Bei den überwiegend männlichen Patienten sind Niere, Gastrointestinaltrakt, Milz, Leber, Pankreas und Herz betroffen (in abnehmender Häufigkeit). Die AA-Amyloidose manifestiert sich binnen 10 bis 20 Jahren nach Erscheinung der Grundkrankheit. Die klinischen Symptome umfassen nephrotische Symtome, Proteinurie oder Kardiomyopathie. Die systemische AA-Amyloidose betrifft auch Nervenzellen. Zur Therpaie der AA-Amyloidose werden eine Reihe von Medikamenten eingesetzt, darunter Alkylanzien, Methotrexat, TNF-α-Blocker, u.a. Auch kann bei terminaler Niereninsuffizienz eine Dialyse oder eine Nierentransplantation erfolgen. Therapeutisches Hauptziel ist es aber, die Synthese des Serum-Amyloid-A -Vorläuferproteins zu inhibieren.

Die AL-Amyloidose kann sich systemisch oder lokal begrenzt manifestieren. Ursächlich zeigt sich eine Erkrankung klonaler Plasmazellen. Dabei werden amyloidogene Leichtketten produziert, welche Fibrillen bilden und sich im Interstitium verschiedener Organe ablagern. Am häufigsten betroffen sind Niere, Herz, Darm, Leber und autonomes Nervensystem. Typisch ist auch das Auftreten einer Polyneuropathie.

AApoAl ist eine proteinöse Komponente von high-density-lipoprotein (HDL), dessen natürliche Funktion es ist, wasserunlösliche Cholesterol und Phospholipide im Blut zu transportieren. Eine Mutation im N-terminalem Bereich, verleiht AApoAI amyloidogene Eigenschaften. Die hereditäre AApoAI-Amyloidose manifestiert sich in einer Polyneuropathie der Füße und Hände. Auch Niere, Herz, Leber (AP und γ-GT erhöht), peripheres Nervensystem, Haut, Larynx und Hoden sind in abnehmender Häufigkeit betroffen.

AApoAII ist eine proteinöse Komponente von high-density-lipoprotein (HDL), dessen natürliche Funktion es ist, wasserunlösliches Cholesterol und Phospholipide im Blut zu transportieren. Eine Mutation des Stop-Codons des ApoAII-Gens, verleiht AApoAII amyloidogene Eigenschaften. Die hereditäre AApoAII-Amyloidose manifestiert sich vorwiegend in Niere und Herz.

Die ATTR-Amyloidose ist die häufigste hereditäre Amyloidose. Sie wird autosomal dominant vererbt. Verantwortlich zeigt sich eine Mutation des Gens für Transthyretin, einem Transportprotein, welches mit dem Ritinol-bindenem Protein und Thyroxin im Plasma assoziiert ist. Die häufigste Mutation Val30Met verleiht dem Protein amyloidogene und somit pathogene Eigenschaften. Im Verlaufe der ATTR-Amyloidose kommt es zu Amyloidablagerungen im peripheren und autonomen Nervensystem. Leitsymptome umfassen erektile Dysfunktion, gastroinestinale Beschwerden, Malabsorption und restriktive Kardiomypathie. Unbehandelt ist die Prognose der ATTR-Amyloidose schlecht. Neben der symptomatischen Behandlung verbleibt als therapeutische Option lediglich eine Lebertransplantation.

In der klinischen Psychiatrie werden bislang alle Erkrankungen einschließlich der Schizophrenie oder der Depression rein klinisch in Form eines Interviews mithilfe eines Fragenkatalogs und ggf. durch Bewegungs- oder Verhaltenstests diagnostiziert. Aufgrund der Unkenntnis über die biologischen Grundlagen von mentalen Erkrankungen stehen keine Blut-oder Liquor-basierten diagnostischen Tests zur Verfügung, wie sie etwa bei neurologischen Erkrankungen verwendet werden, um die klinische Diagnose zu untermauern.

Diese Situation wird besonders von der pharmazeutischen forschenden Industrie als sehr unbefriedigend empfunden, weil die Abwesenheit objektiver und eindeutig quantifizierbarer Tests letztlich quantitatives Monitoring potenziell wirksamer Therapien verhindert.

Durch die Entdeckung von ursächlich wirkenden Krankheitsgenen wie dem DISC1 ist es nun erstmals möglich eine biologische Diagnostik zu entwickeln. DISC1 entwickelt bei einer Subgruppe chronisch mentaler Erkrankungen, wie der Schizophrenie und der rekurrierenden Depression submikroskopische Proteinaggregate im Gehirn. Mithilfe eigens entwickelter Antikörper lassen sich diese im post mortem Gehirn nachweisen.

Die ALS ist eine chronische, schnell voranschreitende Erkrankung des zentralen Nervensystems. Betroffen ist vor allem die willentliche Steuerung der Skelettmuskulatur. Im Verlauf der Krankheit kommt es zu einer Degeneration der für die Muskelbewegung verantwortlichen Motorneuronen. Dies führt zu einer erheblichen Beeinträchtigung bis hin zum völligen Ausfall von Körperbewegungen und -reflexen. Die durchschnittliche Lebenserwartung nach Eintritt der initialen Symptome beträgt nur drei Jahre. Für die ALS sind verschiedene Ätiologien beschrieben worden. Die häufigste Form der ALS ist sporadischer Natur. Es existieren aber auch familiär vererbte ALS-Fälle. Am häufigsten dabei ist eine Mutation der Superoxid Dismutase-1 (SOD1). Neuere Daten legen nahe, dass eine Hexanukleotid-Expansion des Gens C9orf72 ursächlich sowohl für ALS, als auch für Frontotemporaldemenz ist.

Die Diagnose der ALS kann erst spät im Verlauf der Krankheit gestellt werden. Der diagnostische Verzug beläuft sich in der Regel auf ein Jahr nach Einsetzen der ersten Symptome. Die Diagnose der ALS umfasst derzeit eine klinische Begutachtung, eine elektrophysiologische Untersuchung (Elektromyopgraphie) und eine neuropathologische Analyse (Biopsie). Zusätzlich kann eine Liquorpunktion vorgenommen und ein Differentialblutbild erstellt werden. Schließlich muss ALS gegenüber anderen Krankheiten differentialdiagnostisch abgegrenzt werden.

Pathologisches Merkmal der ALS sind unter anderem cytoplasmatische Einschlüsse des Proteins FUS. Bei Zelldegeneration werden diese Aggregate freigesetzt und können so in die Peripherie gelangen, wo sie einer Diagnostik zugänglich sind. Auch andere neurodegenerative Krankheiten zeigen Ablagerungen von FUS-Aggregaten, darunter die Frontotemporale Lobäre Degeneration (FTLD), deren Leitsymptome Veränderungen der Persönlichkeit, des Sozialverhaltens und der sprachlichen Fähigkeiten sind.

Ein alternatives pathologisches Merkmal der ALS sind Ablagerungen des Proteins SOD1 in den Motorneuronen. Bei Zelldegeneration werden diese Aggregate freigesetzt und können so in die Peripherie gelangen, wo sie einer Diagnostik zugänglich sind.

Ein alternatives pathologisches Merkmal der ALS sind cytoplasmatische Einschlüsse des ubiquitinierten Proteins TDP-43. Bei Zelldegeneration werden diese Aggregate freigesetzt und können so in die Peripherie gelangen, wo sie einer Diagnostik zugänglich sind.

Auch andere neurodegenerative Krankheiten gehen einher mit Ablagerungen von TDP-43-Aggregaten, darunter die frontotemporale lobäre Degeneration (FTLD), deren Leitsymptome Veränderungen der Persönlichkeit, des Sozialverhaltens und der sprachlichen Fähigkeiten sind. Neuere Studien sehen auch einen Zusammenhang zwischen TDP-43-Ablagerungen und Chronischer Traumatischer Enzephalopathie (CTE). Von CTE betroffen sind vor allem Leistungssportler und Soldaten, die wiederholt Schädel-Hirn-Traumata erleiden.

Auch bei Diabetes Mellitus Typ II (DMT2) spielen aggregierte, körpereigene Proteine eine Rolle. So findet man z.B. im Pankreas von DMT2-Patienten Plaques, die aus vorwiegend aus dem Insel-Amyloid-Polypeptid (IAPP) bestehen. IAPP ist ein Peptidhormon, das in den beta-Zellen des Pankreas produziert wird und gemeinsam mit Insulin sekretiert wird. Es wird spekuliert, dass die Aggregation des IAPP im Zusammenhang mit dem fortschreitenden Verlust der Insel-beta-Zellen steht.

Bisher sind keine ex vivo Nachweissysteme für IAPP-Oligomere -oder Aggregate in Geweben oder Körperflüssigkeiten im Routineeinsatz.

Die Parkinson-Krankheit ist eine degenerative neurologische Erkrankung. Die Leitsymptome umfassen Muskelstarre, verlangsamte Bewegung, Muskelzittern und Haltungsinstabilität. Darüber hinaus sind fakultative Begleitsymptome, wie z.B. psychische Störungen, beschrieben. Im Verlauf der Krankheit kommt es zum Absterben Dopamin-produzierender Neuronen in der Substantia nigra. Histopathologisch zeigen sich im Gehirngewebe von Parkinson-Patienten zytoplasmatische Einschlüsse, sog. Lewy-Körper, die vorwiegend aus α-Synuclein, Ubiquitin und anderen Proteinablagerungen bestehen.

Tauopathien sind eine Gruppe von neurodegenerativen Krankheiten, die durch eine Anreicherung des Tau-Proteins im Gehirn charakterisiert sind. Durch Hyperphosphorylierung kann das Mikrotubuli-assoziierte Tau in unlösliche Aggregate, sog. neurofibrilläre Bündel, überführt werden. Die Tauopathien umfassen verschiedene Krankheitsbilder wie Morbus Alzheimer, Kortikobasale Degeneration, Silberkornkrankheit, Morbus Pick, FTLD-MAPT (früher: FTDP-17, Frontotemporale Demenz und Parkinsonismus des Chromosom 17), progressive supranukleäre Blickparese, neurofibrilläre Tangle-Demenz, Tauopathie mit glialen Einschlüssen sowie unklassifizierbare Tauopathien. Neuere Studien zählen auch die chronische traumatische Enzephalopathie zu den Tauopathien. Manche der o.a. Krankheiten wie z.B. FTLD-MAPT sind erblich bedingt. Die Ursachen der meisten Tauopathien sind aber noch unbekannt.

Die klinische Präsentation der Tauopathien ist äußerst vielfältig und umfasst Bewegungsstörungen (L-Dopa-sensitive oder atypische Parkinsonsyndrome, Akinesie mit "freezing", supranukleäre Blickparese bis hin zu kortikobasalen Syndromen), Verhaltens- und Sprachstörungen und psychiatrische Symptome wie Aggressivität, psychotisches Zustandsbild und Depression.

Die Huntington-Krankheit ist eine genetische neurodegenerative Krankheit, die die Muskelkoordination betrifft und zur Verminderung der kognitiven Fähigkeiten und psychiatrischen Problemen führt. Die Krankheit betrifft sowohl Männer als auch Frauen und wird durch eine autosomal-dominante Mutation des Huntingtin-Gens bedingt. Eine Expansion eines CAG-Triplet-Repeats führt zu einer Verlängerung des Polyglutamin-Trakts im maturierten Protein, wodurch dieses verstärkt zur Fehlfaltung und Aggregation neigt.

Der familiäre viszeralen Amyloidose liegt eine Mutation des Gens für Lysozym zugrunde, welche dem maturierten Protein amyloidogene Eigenschaften verleiht. Amyloidablagerungen finden sich systemisch in einer Vielzahl von Organen.

Krankheitsindikatoren, also Indikatoren zur Erkennung von Krankheiten, auch Biomarker genannt, wurden schon immer zur Diagnose von Krankheiten herangezogen. Bekannte Beispiele für Biomarker sind z.B. der Gehalt von Glukose im Urin als Hinweis auf Diabetes Mellitus oder der Gehalt an HCG im Urin in Schwangerschaftstests.

Die WO 2008/070229 A2 offenbart ein Verfahren zum Nachweis pathogener Aggregate eines Proteins in einer Probe.

S. HÜBINGER ET AL offenbaren in "Detection of α-Synuclein Aggregates by Fluorescence Microscopy", REJUVENATION RESEARCH, Bd. 15, Nr. 2, 1. April 2012 (2012-04-01), Seiten 213-216 einen Ansatz zur Quantifizierung einzelner α-Synuclein-Aggregate als möglicher Biomarker für Parkinson.

Die WO 2007/126473 A2 offenbart Verfahren und dementsprechend in vitro-Systeme zur Erzeugung stabiler und löslicher Oligomere von Amyloidproteinen bei physiologischem pH und physiologischer Temperatur.

Die US 2008/220449 A1 offenbart Verfahren, Zusammensetzungen und Systeme zum Diagnostizieren, Stratifizieren oder Überwachen der Progression oder Regression der Alzheimer-Krankheit.

TOKUDA T ET AL offenbaren in "Detection of elevated levels of alpha-synuclein oligomers in CSF from patients with Parkinson disease", NEUROLOGY, LIPPINCOTT WILLIAMS & WILKINS, PHILADELPHIA, US, Bd. 75, Nr. 20, 1. November 2010 (2010-11-01), Seiten 1766-1772, dass der Gehalt an α-Synuclein-Oligomeren im Liquor und das Verhältnis von Oligomeren zu Gesamt-α-Synuclein nützliche Biomarker für die Diagnose und Früherkennung von Parkinson sein können.

VOLLES ET AL offenbaren in "Relationships between the Sequence of α-Synuclein and its Membrane Affinity, Fibrillization Propensity, and Yeast Toxicity", JOURNAL OF MOLECULAR BIO, ACADEMIC PRESS, UNITED KINGDOM, Bd. 366, Nr. 5, 9. Februar 2007 (2007-02-09), Seiten 1510-1522, dass Beta-Synuclein eine mittlere Toxizität für Hefe aufweist und Gamma-Synuclein nicht toxisch ist.

MICHAEL R. SIERKS ET AL offenbaren in "CSF levels of oligomeric alpha-synuclein and beta-amyloid as biomarkers for neurodegenerative disease", INTEGRATIVE BIOLOGY, Bd. 3, Nr. 12, 10. November 2011 (2011-11-10), Seiten 1188-1196, dass der Nachweis spezifischer oligomerer Aggregatspezies vielversprechend als empfindliche Biomarker für neurodegenerative Erkrankungen ist.

FUNKE ET AL offenbaren in "Single particle detection of Abeta aggregates associated with Alzheimer's disease", BIOCHEMICAL AND BIOPHYSICAL RESEARCH COMMUNICATIONS, ACADEMIC PRESS INC. ORLANDO, FL, US, Bd. 364, Nr. 4, 24. Oktober 2007 (2007-10-24), Seiten 902-907 eine klare Unterscheidung zwischen an Alzheimer erkrankten Personen und nicht dementen Kontrollpersonen durch die Analyse von Liquor cerebrospinalis (Liquor) mittels konfokaler Abtastung der an der Oberfläche erfassten Abeta-Aggregate und anschließender zweidimensionaler Analyse der Fluoreszenzintensitätsverteilung.

FUNKE ET AL offenbaren in "An Ultrasensitive Assay for Diagnosis of Alzheimer's Disease", REJUVENATION RESEARCH, Bd. 11, Nr. 2, 1. April 2008 (2008-04-01), Seiten 315-318 einen ultrasensitiver Test für die frühe und nicht-invasive Diagnose von Alzheimer.

FUNKE ET AL offenbaren in "Single-Particle Detection System for Ab Aggregates: Adaptation of Surface-Fluorescence Intensity Distribution Analysis to Laser Scanning Microscopy", REJUVENATION RESEARCH, Bd. 13, Nr. 2-3, 4. Dezember 2009 (2009-12-04), Seiten 206-209, dass Oberflächen-FIDA auch mit einem Laser-Scanning-Mikroskop durchgeführt werden kann und dann sehr gut für die Charakterisierung von Aß-Aggregaten geeignet ist

WANG-DIETRICHET ET AL offenbaren in "The amyloid-beta oligomer count in cerebrospinal fluid is a biomarker for Alzheimer's disease", JOURNAL OF ALZHEIMER'S DISEASE, Bd. 34, 11. Januar 2013 (2013-01-11), Seiten 985-994, dass die Anzahl der Aβ-Oligomere in Körperflüssigkeiten der direkteste und relevanteste Biomarker für Alzheimer ist.

Pilar Infiesta ET AL offenbaren in "Development of an assay to measure tau oligomer levels in AD", 42nd Annual Meeting of the Society-for-Neuroscience; New Orleans, LA, USA, 17. Oktober 2012 (2012-10-17) den Nachweis von Tau-Aggregaten in CSF.

Pedersen Jeppe T. ET AL offenbaren in "Analysis of Protein Aggregation in Neurodegenerative Disease", Analytical Chemistry, Bd. 85, Nr. 9, 7. Mai 2013 (2013-05-07), Seiten 4215-4227 einen Überblick über etablierte und neue Schlüsseltechniken für die Analyse von Proteinaggregation in frühen, mittleren und späten Stadien, geordnet oder ungeordnet, mit Schwerpunkt auf Beispielen aus der Analyse von Proteinen, die an neurodegenerativen Erkrankungen beteiligt sind.

Die US 2009/042211 A1 offenbart ein Verfahren zum selektiven Nachweis des Vorliegens und/oder der Menge von pathologischen Proteinablagerungen.

Takashi Kasai ET AL offenbaren in "Utilization of a multiple antigenic peptide as a calibration standard in the BAN50 single antibody sandwich ELISA for A[beta] oligomers", Biochemical and Biophysical Research Communications, Bd. 422, Nr. 3, 1. Juni 2012 (2012-06-01), Seiten 375-380, dass die aus einem 16-mer MAP erstellte Standardkurve den physiologischen Bereich der Signale abdeckt, die im BAN50 SAS-ELISA aus Proben von menschlichem Liquor, Serum und Plasma erhalten wurden.

EL-AGNAF OMAR M A ET AL offenbaren in "Detection of oligomeric forms of α-synuclein protein in human plasma as a potential biomarker for Parkinson's disease", FASEB JOURNAL" Bd. 20, Nr. 3, 1. März 2006 (2006-03-01), Seiten 419-425 neue Möglichkeiten für die Entwicklung von Diagnosetests für Parkinson und verwandte Krankheiten sowie für die Erprobung von Therapeutika zur Verhinderung oder Umkehrung der Aggregation von alphasyn.

Um eine immer größer und älter werdende Bevölkerung medizinisch zu versorgen, ist die Identifikation von neuen, spezifischen Biomarkern notwendig, die eine sichere Diagnose gewährleisteten. Eine hohe Spezifität ist insbesondere bei Krankheiten mit ähnlichen Symptomen und Krankheitsbildern notwendig, um hier eine erfolgversprechende Behandlung durchführen zu können. Ferner sollen die Krankheitsindikatoren Risikopersonen schon in einem frühen Krankheitsstadium oder im Falle eines Rückfalls möglichst bald identifizieren. Die Biomarker sollen in leicht zugänglichem Biomaterial vorhanden und identifizierbar sein und einen schnellen Nachweis ermöglichen. Dadurch ist es nicht nur möglich, den Verlauf der Krankheit, sondern auch die Wirkung der Behandlung und der Vorbeugung zu verfolgen.

Aufgabe der vorliegenden Erfindung war es, solche Biomarker zur Verfügung zu stellen, insbesondere für Krankheiten, die Aggregate fehlgefalteter Proteine aufweisen.

Weitere Aufgabe war es, ein schnelles und sicheres Verfahren zu deren selektiven Quantifizierung und/oder Charakterisierung zur Verfügung zu stellen. Insbesondere sollen die Krankheitsindikatoren für den klinischen Routineeinsatz geeignet sein. Dies bedeutet, dass sie in einem, bevorzugt Schnelltest und ggf. mittels Standards charakterisiert werden können, so dass vergleichbare und unabhängige Werte bestimmt werden.

Ferner sollten die o.g. Anforderungen an Biomarker erfüllt werden und die entsprechenden, gegenüber dem Stand der Technik verbesserten, Möglichkeiten und Werkzeuge zur Verfügung gestellt werden.

Die Diagnose der AA-Amyloidose umfasst eine Begutachtung der klinischen Präsentation. Problematisch ist dabei aber die differentialdiagnostische Abgrenzung gegenüber sehr vielen anderen Krankheitsbildern. Präklinisch ist die Diagnose nur durch den histologischen Nachweis des AA-Amyloids mittels Kongorotfärbung möglich. Dazu ist aber eine Fettaspirations-Biopsie erforderlich. Eine Routinediagnostik, welche die amyloiden Proteinaggregate als Biomarker in Körperflüssigkeiten nachweist, ist gegenwärtig nicht etabliert.

Die AA-Amyloidose ist gekennzeichnet durch Fehlfaltung des Serum-Amyloid-A - Vorläuferproteins (AA). Die AA-Aggregate könnten somit ein direkter Biomarker für die AA-Amyloidose sein. Der diagnostische Nachweis stellt indes eine technische Herausforderung dar, da AA auch im gesunden Organismus ubiquitär produziert wird. Daher muss das Verfahren insensitiv gegenüber einem hohen Überschuss an normal gefaltetem, monomerem AA sein.

Die Diagnose der AL-Amyloidose umfasst eine Begutachtung der klinischen Präsentation, welche sich aber stark variabel zeigen kann. Problematisch ist darüber hinaus die differentialdiagnostische Abgrenzung gegenüber sehr vielen anderen Krankheitsbildern. Goldstandard ist der histologische Nachweis des AL-Amyloids mittels Kongorotfärbung. Zusätzlich sollte die Immunhistochemie durchgeführt werden. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die AL-Amyloidose ist gekennzeichnet durch Fehlfaltung des IgG-light-chain-Vorläuferproteins (AL). Die AL-Aggregate könnten somit ein direkter Biomarker für die AL-Amyloidose sein. Der diagnostische Nachweis stellt indes eine technische Herausforderung dar, da AL auch im gesunden Organismus ubiquitär produziert wird. Daher muss das Verfahren insensitiv gegenüber einem hohen Überschuss an normal gefaltetem, monomerem AL sein.

Die Diagnose der AApoAI-Amyloidose umfasst eine Begutachtung der klinischen Präsentation. Problematisch ist dabei aber die differentialdiagnostische Abgrenzung gegenüber sehr vielen anderen Krankheitsbildern. Goldstandard ist der histologische Nachweis des AApoAI-Amyloids mittels Kongorotfärbung. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die AApoAI-Amyloidose ist gekennzeichnet durch Fehlfaltung des Apolipoprotein AI-Vorläufers (AApoAI). Die AApoAI-Aggregate könnten somit ein direkter Biomarker für die AApoAI-Amyloidose sein. Der diagnostische Nachweis stellt indes eine technische Herausforderung dar, da AApoAI auch im gesunden Organismus ubiquitär produziert wird. Daher muss das Verfahren insensitiv gegenüber einem hohen Überschuss an normal gefaltetem, monomerem AApoAI sein.

Die Diagnose der AApoAII-Amyloidose umfasst eine Begutachtung der klinischen Präsentation. Problematisch ist dabei aber die differentialdiagnostische Abgrenzung gegenüber sehr vielen anderen Krankheitsbildern. Goldstandard ist der histologische Nachweis des AApoAII-Amyloids mittels Kongorotfärbung. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die AApoAII-Amyloidose ist gekennzeichnet durch Fehlfaltung des Apolipoprotein All-Vorläufers (AApoAII). Die AApoAII-Aggregate könnten somit ein direkter Biomarker für die AApoAII-Amyloidose sein. Der diagnostische Nachweis stellt indes eine technische Herausforderung dar, da AApoAll auch im gesunden Organismus ubiquitär produziert wird. Daher muss das Verfahren insensitiv gegenüber einem hohen Überschuss an normal gefaltetem, monomerem AApoAII sein.

Die Diagnose der ATTR-Amyloidose umfasst eine Begutachtung der klinischen Präsentation. Problematisch ist dabei aber die differentialdiagnostische Abgrenzung gegenüber sehr vielen anderen Krankheitsbildern. Zudem sind Zeitpunkt der Erstsymptomatik, Verlauf der Erkrankung und Phänotyp regional unterschiedlich. Goldstandard ist der histologische Nachweis des ATTR-Amyloids mittels Kongorotfärbung. Dazu ist aber eine aufwendige Biopsie erforderlich. Eine Routinediagnostik, welche die amyloiden Proteinaggregate als Biomarker in Körperflüssigkeiten nachweist, ist gegenwärtig nicht etabliert.

Die ATTR-Amyloidose ist gekennzeichnet durch Fehlfaltung des Transthyretin-Vorläuferproteins (TTR). Die ATTR-Aggregate könnten somit ein direkter Biomarker für die ATTR-Amyloidose sein. Der diagnostische Nachweis stellt indes eine technische Herausforderung dar, da ATTR auch im gesunden Organismus ubiquitär produziert wird. Daher muss das Verfahren insensitiv gegenüber einem hohen Überschuss an normal gefaltetem, monomerem ATTR sein.

Schizophrene Psychosen können gegenwärtig erst drei bis fünf Jahre nach dem Auftreten der ersten Symptome diagnostiziert werden. Eine Diagnostik, die auf dem Nachweis eines Biomarkers beruht, ist für chronisch mentale Erkrankungen wie die Schizophrenie bislang nicht im Routineeinsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Neuere Studien sehen einen Zusammenhang zwischen chronisch mentalen Erkrankungen, wie der Schizophrenie oder der rekurrierenden Depression, und aggregiertem DISC1-Protein (disrupted-in-schizophrenia-1). Diese DISC1-Aggregate könnten ein direkter Biomarker für Schizophrenie, rekurrierende Depression und andere DISC1opathien sein. Der diagnostische Nachweis stellt indes eine technische Herausforderung dar, da DISC1 auch im gesunden Organismus produziert wird. Daher muss das Verfahren insensitiv gegenüber einem hohen Überschuss an normal gefaltetem, monomerem DISC1 sein.

Eine Diagnose, die auf dem Nachweis eines Biomarkers beruht, ist sowohl für ALS als auch für FTLD gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Bei der Amyotrophen Lateralsklerose (ALS) und der Frontotemporalen Lobärdegeneration (FTLD) wurden cytoplasmatische Ablagerungen des RNA-Bindeproteins FUS (Fused in Sarcoma) beschrieben. Diese FUS-Aggregate könnten ein direkter Biomarker für die beschriebenen Krankheitsbilder sein. Der diagnostische Nachweis stellt indes eine technische Herausforderung dar, da FUS auch im gesunden Organismus ubiquitär produziert wird. Daher muss das Verfahren insensitiv gegenüber einem hohen Überschuss an normal gefaltetem, monomerem FUS sein.

Gegenwärtig gilt der Blutzuckerspiegel als allgemein anerkannter Biomarker für DMT2, womit eine sichere Diagnose für Patienten erreicht werden kann. Wird der Blutzuckerspiegel mit Hilfe des Insulins reguliert, ist ein Nachweis von DMT2 nicht mehr möglich. Dies hat eine besondere Bedeutung für die Analyse von Blutkonserven, die für Transfusionen eingesetzt werden, da diese möglicherweise ein Gefährdungspotenzial für die Empfänger bedeuten. Auch für eine ursächliche Therapieentwicklung von DMT2 ist die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Seit einigen Jahren wird über einen Zusammenhang zwischen Diabetes Mellitus Typ 2 (DMT2) und der Alzheimerschen Demenz (AD) berichtet. Studien zufolge sind rund 80 % der AD-Patienten an DMT2 erkrankt bzw. weisen einen höheren Glucose-Gehalt im Blut auf. Zudem zeigen die DMT2 und AD Mäuse ähnliche verhaltensbezogene, kognitive und vaskuläre Anomalien. Beide Krankheiten weisen ähnliche pathologische Merkmale auf: die Entstehung von amyloiden Plaques im Pankreas (DMT2) oder im Gehirn (AD) infolge von Proteinfaltung und darauf folgenden Fibrillenbildung des IAPP- bzw. Aß-Peptids. Insbesondere die kleinen Aggregate gelten als hauptsächlicher Verursacher für die Entstehung und den Fortschritt von DMT2 und AD. IAPP-Aggregate könnten daher ein direkter Biomarker für DMT2 sein. Der diagnostische Nachweis stellt indes eine technische Herausforderung dar, da IAPP auch im gesunden Organismus ubiquitär produziert wird. Daher muss das Verfahren insensitiv gegenüber einem hohen Überschuss an normal gefaltetem, monomerem IAPP sein.

Eine Diagnose, die auf dem Nachweis eines Biomarkers beruht, ist für ALS gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist wohl das Auftreten von fehlgefalteten, aggregierten Proteinen im Krankheitsverlauf. Bei der Amyotrophen Lateralsklerose (ALS) wurden Ablagerungen der Superoxiddismutase 1 (SOD1) beschrieben. Diese SOD1-Aggregate könnten ein direkter Biomarker für ALS sein. Der diagnostische Nachweis stellt indes eine technische Herausforderung dar, da SOD1 auch im gesunden Organismus ubiquitär produziert wird. Daher muss das Verfahren insensitiv gegenüber einem hohen Überschuss an normal gefaltetem, monomerem SOD1 sein.

Die Diagnose der Parkinson-Krankheit wird durch den sog. L-Dopa-Test geführt. Dabei wird dem Patienten L-Dopa in Kombination mit einem Decarboxylasehemmer verabreicht. Führt diese Behandlung zu einer deutlichen Verbesserung der Symptome, kann mit sehr hoher Wahrscheinlichkeit die Parkinson-Krankheit diagnostiziert werden.

Eine Diagnostik, die auf einem minimalinvasiven Nachweis eines Biomarkers in Körperflüssigkeiten beruht, ist für die Parkinson-Krankheit aber bislang nicht im Routineeinsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist wohl das Auftreten von fehlgefalteten, aggregierten Proteinen im Krankheitsverlauf. Die Parkinson-Krankheit ist gekennzeichnet durch pathologische Akkumulation von aggregiertem α-Synuclein. Diese α-Synuclein-Aggregate könnten ein direkter Biomarker für die Parkinson-Krankheit und anderer α-Synucleinopathien sein. Der diagnostische Nachweis stellt indes eine technische Herausforderung dar, da α-Synuclein auch im gesunden Organismus produziert wird. Daher muss das Verfahren insensitiv gegenüber einem hohen Überschuss an normal gefaltetem, monomerem α-Synuclein sein.

Der neuropathologischen Phänotyp der verschiedenen Tauopathien wird post mortem identifiziert. Eine Schwierigkeit stellt die erhebliche Überschneidung verschiedener neuropathologischer Veränderungen dar. Darüber hinaus liegen diverse Pathologien oft in Kombination vor, was die Notwendigkeit der Biomarkerforschung unterstreicht. Eine Diagnose, die auf dem Nachweis eines Biomarkers in Körperflüssigkeiten beruht, ist für Tauopathien gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist wohl das Auftreten von fehlgefalteten, aggregierten Proteinen im Krankheitsverlauf. Tauopathien sind gekennzeichnet durch pathologische Ablagerungen des Tau-Proteins. Diese Tau-Aggregate könnten ein direkter Biomarker für verschiedene Krankheitsbilder sein. Der diagnostische Nachweis stellt indes eine technische Herausforderung dar, da Tau auch im gesunden Organismus ubiquitär produziert wird. Daher muss das Verfahren insensitiv gegenüber einem hohen Überschuss an normal gefaltetem, monomerem Tau sein.

Eine Diagnose, die auf dem Nachweis eines Biomarkers beruht, ist sowohl für ALS als auch für FTLD und CTE gegenwärtig nicht im Routineeinsatz. Ebenso gibt es zum jetzigen Zeitpunkt keine ursächliche Therapie. Lediglich neuroprotektive und symptomatische Therapien sind im Einsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für genetisch bedingte Fälle, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Bei der Amyotrophen Lateralsklerose (ALS) und der Frontotemporalen Lobärdegeneration (FTLD) wurden Ablagerungen des TAR DNA-Bindeproteins 43 (TDP-43) beschrieben. Diese TDP-43-Aggregate könnten ein direkter Biomarker für die beschriebenen Krankheitsbilder sein. Der diagnostische Nachweis stellt indes eine technische Herausforderung dar, da TDP-43 auch im gesunden Organismus ubiquitär produziert wird. Daher muss das Verfahren insensitiv gegenüber einem hohen Überschuss an normal gefaltetem, monomerem TDP-43 sein.

Bei Verdacht auf die Huntington-Krankheit kann ein Gentest durchgeführt werden, der die Krankheit mit Sicherheit feststellen kann. Eine Diagnostik, die auf einem minimalinvasiven Nachweis eines Biomarkers in Körperflüssigkeiten beruht, ist für die Huntington-Krankheit aber bislang nicht im Routineeinsatz. Dabei ist für eine Therapieentwicklung die Identifikation und Standardisierung eines Biomarkers im frühen oder gar präsymptomatischen Krankheitsverlauf von immenser und vermutlich entscheidender Wichtigkeit. Dies gilt auch für die genetisch bedingte Huntington-Krankheit, da auch hier der Krankheits- und Therapieverlauf verfolgt werden kann.

Charakteristisch für eine Vielzahl von Erkrankungen des zentralen Nervensystems ist das Auftreten von fehlgefalteten, aggregierten Proteinen im Krankheitsverlauf. Die Huntington-Krankheit ist gekennzeichnet durch pathologische Akkumulation von aggregiertem Huntingtin. Diese Huntingtin-Aggregate könnten ein direkter Biomarker für die Huntington-Krankheit sein. Der diagnostische Nachweis stellt indes eine technische Herausforderung dar, da Huntingtin auch im gesunden Organismus produziert wird. Daher muss das Verfahren insensitiv gegenüber einem hohen Überschuss an normal gefaltetem, monomerem Huntingtin sein.

Die Diagnose der ALys-Amyloidose umfasst eine Begutachtung der klinischen Präsentation. Problematisch ist dabei aber die differentialdiagnostische Abgrenzung gegenüber sehr vielen anderen Krankheitsbildern. Goldstandard ist der histologische Nachweis des ALys-Amyloids mittels Kongorotfärbung und Immunohistochemie. Dazu ist aber eine aufwendige Biopsie erforderlich. Eine Routinediagnostik, welche minimalinvasiv die amyloiden Proteinaggregate als Biomarker in Körperflüssigkeiten nachweist, ist gegenwärtig nicht etabliert.

Die familiäre viszerale Amyloidose ist gekennzeichnet durch Fehlfaltung des Lysozym-Proteinvorläufers (ALys). Die ALys-Aggregate könnten somit ein direkter Biomarker für die ALys-Amyloidose sein. Der diagnostische Nachweis stellt indes eine technische Herausforderung dar, da Lysozym auch im gesunden Organismus ubiquitär produziert wird. Daher muss das Verfahren insensitiv gegenüber einem hohen Überschuss an normal gefaltetem, monomerem ALys sein.

Aufgabe der vorliegenden Erfindung war es auch, ein Routineverfahren zur quantitativen Bestimmung von Krankheitsindikatoren zur Verfügung zu stellen, insbesondere von mehreren Krankheitsindikatoren nebeneinander in derselben Probe. Ferner sollte das Verfahren die Möglichkeit bieten, die Probe mit nur wenigen oder bevorzugt keinen Aufarbeitungsschritten zu analysieren.

Eine Vielzahl von neurodegenerativen Krankheiten und Amyloidosen ist gekennzeichnet durch das simultane Auftreten verschiedener aggregierter Proteinspezies. Als Beispiel sei die Alzheimer'sche Demenz angeführt, bei der sowohl Ablagerungen von Amyloid beta als aus Tau zu beobachten sind (Jucker and Walker, Annals of neurology 70, 532-540, 2011; Spillantini and Goedert, Lancet neurology 12, 609-622, 2013; die unter Bezugnahme eingeschlossen sind). Bei der frontotemporalen Lobärdegeneration (FTLD) koexistieren Tau und TDP-43 als pathologische Proteinaggregate (Mackenzie et al., Acta neuropathologica 119, 1-4, 2010). TDP-43-Pathologie manifestiert sich wiederum auch in der Amyotrophen Lateralsklerose und Chronischen Traumatischen Enzephalopathie (Blennow et al., Neuron 76, 886-899, 2012; Blokhuis et al., Acta neuropathologica 125, 777-794, 2013; die unter Bezugnahme eingeschlossen sind).

Das Verfahren muss eine ausreichende Spezifität sicherstellen, um unterschiedliche Aggregat-Typen nebeneinander und/oder gleichzeitig zu untersuchen. Somit war es auch Aufgabe der Erfindung, ein Verfahren mit gleichzeitig hoher Spezifität für die Untersuchung unterschiedlicher Aggregat-Typen zu bieten, und so Störungen der Untersuchung eines Aggregat-Typs durch einen zweiten Aggregat-Typ auszuschließen. Es sollen auch Aggregat-Mischformen, bestehend aus verschiedenen Protein-Monomeren untersucht werden können.

Die bisher beschriebenen Protein-Aggregate der o.g. Krankheiten stellen lediglich ein pathologisches Hauptmerkmal des jeweiligen Krankheitsbildes dar. Ihre Verwendung als Biomarker ist nicht sichergestellt.

Diese Aufgabe wird gelöst durch ein Verfahren zur qualitativen und/oder quantitativen Bestimmung von Krankheitsindikatoren gemäß Anspruch 1.

Erfindungsgemäß ist das Verfahren dadurch gekennzeichnet, dass die Probe ohne weitere Aufarbeitung und/oder Behandlung nach der Untersuchung auf einen ersten Aggregat-Typ auf mindestens einen weiteren, zum Beispiel zweiten unterschiedlichen Aggregat-Typen körpereigener fehlgefalteter Proteine untersucht wird.

In einer Ausführung ist das Verfahren dadurch gekennzeichnet, dass die Probe auf mindestens zwei unterschiedliche Aggregat-Typen in einem Verfahrensschritt untersucht wird.

In einer weiteren Ausführung ist das Verfahren dadurch gekennzeichnet, dass die Probe auf mindestens zwei unterschiedliche Aggregat-Typen auf demselben Substrat untersucht wird. Erfindungsgemäß ist das Verfahren, dadurch gekennzeichnet, dass der Aggregat-Typ körpereigener fehlgefalteter Proteine ausgewählt wird aus der Gruppe bestehend aus α-Synuclein-Aggregate und Tau-Aggregate.

Erfindungsgemäß ist das Verfahren dadurch gekennzeichnet, dass die Detektion in Schritt c) mittels einem Verfahren mit ortsaufgelöstem Signal, bevorzugt sFIDA-Verfahren erfolgt.

Erfindungsgemäß ist das Verfahren dadurch gekennzeichnet, dass vor Schritt a) eine Immobilisierung von Fängermolekülen auf dem Substrat erfolgt.

In einer weiteren Ausführung ist das Verfahren dadurch gekennzeichnet, dass die Fängermoleküle und/oder die Sonde mit Fluoreszenzfarbstoffen gekennzeichnet sind.

In einer weiteren Ausführung ist das Verfahren dadurch gekennzeichnet, dass die Fängermoleküle und/oder die Sonden spezifische Anti-Körper gegen ein Epitop der Proteine aufweisen, die die Aggregate bilden.

In einer weiteren Ausführung ist das Verfahren dadurch gekennzeichnet, dass ein interner oder externer Standard eingesetzt wird.

Gegenstand der Erfindung ist auch die Verwendung eines Standards zur selektiven Quantifizierung und/oder Charakterisierung der Krankheitsindikatoren in einem Verfahren, wie vorstehen beschrieben, wobei es sich bei dem Standard um ein Dendrimer handelt, welches ein zentrales Gerüstmolekül und Epitope der Proteine α-Synuclein-Aggregate oder Tau-Aggregate gemäß SEQ ID NO: 10 oder SEQ ID NO: 11 enthält, wobei sowohl Standards, die aus den gleichen Monomer-Sequenzen aufgebaut sind, als auch solche, die aus unterschiedlichen Monomer-Sequenzen aufgebaut sind verwendet werden.

Solche Standard-Moleküle können zur Inaktivierung der jeweiligen Protein-Aggregate verwendet werden, zum Beispiel durch Binden oder Zerstören der Protein-Aggregate, oder prophylaktisch, um die Bildung der Protein-Aggregate zu verhindern, was jedoch nicht Gegenstand der vorliegenden Erfindung ist. Ferner ist Gegenstand der Erfindung ein Verfahren zur Bestimmung von Krankheiten die Aggregate fehlgefalteter Proteine aufweisen, umfassend folgende Schritte:
i) quantitativen Bestimmung ex vivo von Krankheitsindikatoren bestimmt in dem erfindungsgemäßen Verfahren;
ii) Vergleich dieser Daten mit den Normwerten;
iii) Feststellen einer signifikant unterschiedlichen Menge Krankheitsindikatoren bei diesem Vergleich;
iv) Zuordnung der Abweichung zu einer Krankheit ausgewählt aus der Gruppe bestehend aus bestehend aus Tauopathien, Parkinson-Krankheit und andere Synucleinopathien und/oder Alzheimer'sche Demenz.

Unter dem Begriff "Aggregat-Typ" ist im Sinne der Erfindung ein Aggregat einer bestimmten, definierten Zusammensetzung körpereigener fehlgefalteter Proteine zu verstehen. In einer Alternative ist ein Aggregat-Typ aus gleichen Peptiden (Monomeren) zusammengesetzt (aufgebaut), d.h. aus Peptiden derselben Aminosäure-Sequenz oder aus unterschiedlichen Homologen ein und desselben Peptids. In anderen Alternative ist ein Aggregat-Typ aus unterschiedlichen Peptiden (Monomeren) zusammengesetzt, aufgebaut, d.h. aus Peptiden unterschiedlicher Aminosäure-Sequenz oder aus Homologen der unterschiedlichen Peptide. In einer Probe können auch beide Alternativen vorhanden sein.

In einer Ausführung der vorliegenden Erfindung bedeutet die "qualitative Bestimmung von Krankheitsindikatoren" die Identifikation von neuen, spezifischen Biomarkern. Mit anderen Worten, es werden Krankheitsindikatoren identifiziert und neu definiert. Erfindungsgemäß sind Krankheitsindikatoren durch die Anwesenheit, gegebenenfalls in einer bestimmten Konzentration, und/oder Abwesenheit jeweils eines oder mehrerer Aggregate körpereigener fehlgefalteter Proteine definiert.

In einer weiteren Ausführung bedeutet die "qualitative Bestimmung von Krankheitsindikatoren" die Überprüfung bzw. Bestimmung der An- und/oder Abwesenheit dieser Biomarker.

Im Sinne der vorliegenden Erfindung bedeutet die "quantitative Bestimmung von Krankheitsindikatoren" in einer ersten Alternative die Bestimmung der Konzentration der Biomarker. In einer zweiten Alternative wird Zusammensetzung, Größe und/oder Form der Biomarker bestimmt. Es können auch beide Alternativen, bevorzugt gleichzeitig, in einem Verfahrensschritt) durchgeführt werden.

Die quantitative Bestimmung von Krankheitsindikatoren ist der selektiven Quantifizierung und/oder Charakterisierung von Aggregat-Typen als Biomarker äquivalent. Sobald die Merkmale eines Krankheitsindikators definiert sind, erfolgt seine quantitative Bestimmung, d.h. seine selektiven Quantifizierung: also Konzentrationsbestimmung die die Information über An- bzw. Abwesenheit enthält; und/oder seine Charakterisierung: also Bestimmung der Zusammensetzung, Größe und/oder Form.

Erfindungsgemäß wird eine Probe auf mindestens zwei Aggregat-Typen körpereigener fehlgefalteter Proteine untersucht. In einer Alternative der vorliegenden Erfindung wird eine Probe auf mindestens drei Aggregat-Typen körpereigener fehlgefalteter Proteine untersucht. In einer Alternative der vorliegenden Erfindung wird eine Probe auf mindestens vier Aggregat-Typen körpereigener fehlgefalteter Proteine untersucht. In einer Alternative der vorliegenden Erfindung wird eine Probe auf mindestens fünf Aggregat-Typen körpereigener fehlgefalteter Proteine untersucht. In einer Alternative der vorliegenden Erfindung wird eine Probe auf mindestens sechs Aggregat-Typen körpereigener fehlgefalteter Proteine untersucht. In einer Alternative der vorliegenden Erfindung wird eine Probe auf mindestens sieben Aggregat-Typen körpereigener fehlgefalteter Proteine untersucht. In einer Alternative der vorliegenden Erfindung wird eine Probe auf mindestens acht Aggregat-Typen körpereigener fehlgefalteter Proteine untersucht. In einer Alternative der vorliegenden Erfindung wird eine Probe auf mindestens neun Aggregat-Typen körpereigener fehlgefalteter Proteine untersucht. In einer Alternative der vorliegenden Erfindung wird eine Probe auf mindestens zehn Aggregat-Typen körpereigener fehlgefalteter Proteine untersucht. In einer Alternative der vorliegenden Erfindung wird eine Probe auf mindestens 11, 12 ,13 ,14 ,15 oder mehr Aggregat-Typen körpereigener fehlgefalteter Proteine untersucht.

In einer Ausführung der vorliegenden Erfindung sind die Aggregate aus Peptiden bzw. Monomeren der SEQ ID NO: 10 oder11:
SEQ ID NO: 10; Tau-Aggregate : SEQ ID NO: 11.

Es können jedoch auch gemischte Aggregate vorliegen, aufgebaut aus mindestens zwei unterschiedlichen Proteinen, Monomeren, sogenannte Aggregat-Mischformen.

Der Begriff " Aggregat-Typ körpereigener fehlgefalteter Proteine untersuchen" bzw. Synonyme wie zum Beispiel "Untersuchung" davon bedeuten im Sinne der Erfindung die qualitative und/oder quantitative Bestimmung (Analyse) des jeweiligen Aggregat-Typs. Bei der qualitativen Bestimmung wird die An- bzw. Abwesenheit bestimmt während bei der quantitativen Bestimmung Konzentration, Zusammensetzung, Größe und/oder Form der bestimmt wird.

Der Begriff "ohne weitere Aufarbeitung und/oder Behandlung" der Probe zur Untersuchung auf einen zweiten Aggregat-Typ bedeutet erfindungsgemäß in einer Alternative, dass zwischen zwei Untersuchungen auf unterschiedliche Aggregat-Typen keine Aufarbeitung und/oder Behandlung der Probe stattfindet. In einer anderen Alternative findet in der Probe nach Untersuchung eines ersten oder eines weiteren Aggregat-Typs keine chemischen Reaktionen statt. Bevorzugt erfolgt die erste und zweite und gegebenenfalls jede weiter Untersuchung in einem Probenaliquot. Die Probe erfährt gegebenenfalls lediglich physikalische oder mechanische Einwirkungen wie zum Beispiel Temperaturänderung oder Pipettieren. In einer weiteren oder zusätzlichen Alternative erfährt die Probe keine physikalische oder mechanische Einwirkungen. In einer weiteren oder zusätzlichen Alternative hat die Probe zur Untersuchung eines zweiten Aggregat-Typs dieselbe chemische Zusammensetzung wie die Probe zur Untersuchung des ersten Aggregat-Typs, gegebenenfalls sind dieselben Verbindungen anwesend jedoch in geänderten Konzentrationen. Außerdem enthält die Probe zur Untersuchung eines zweiten Aggregat-Typs gegebenenfalls die Fängermoleküle, Sonden und/oder Standards zur Untersuchung des ersten Aggregat-Typs sowie gegebenenfalls die entsprechenden Verbindungen aus Aggregat, Fängermolekül und/oder Sonde. In einer weiteren oder zusätzlichen Alternative werden die Sonden für die Untersuchung auf einen zweiten Aggregat-Typ erst nach der Untersuchung auf den ersten Aggregat-Typ dazu gegeben, so dass als einzige chemische Reaktion die Bindung der spezifischen Sonde für den zweiten Aggregat-Typ an dieses Aggregat ist, sofern in der Probe enthalten.

Analog gilt der oben beschriebene Sachverhalt für die Untersuchung eines dritten, vierten, fünften, sechsten, siebenten, achten, neunten, zehnten und/oder weiteren Aggregat-Typs.

Bei den Biomarkern, auch Krankheitsindikatoren genannt, handelt es sich erfindungsgemäß in Anlehnung an die Definition des NIH um Parameter, mit denen sich eine Eigenschaft objektiv messen lässt, um sie als Indikator für normale biologische, pathogene Prozesse oder pharmakologische Antworten auf eine therapeutische Intervention heranzuziehen. Die erfindungsgemäß bestimmten und verwendeten Krankheitsinhibitoren sind mit physikalischen und/oder chemischen Verfahren qualitativ und quantitativ zu charakterisieren. Aggregate im Sinne der vorliegenden Erfindung sind
- Partikel, die aus mehreren, bevorzugt gleichen Bausteinen bestehen, die nicht kovalent miteinander verbunden sind und/oder
- nicht-kovalente Zusammenlagerungen mehrerer Monomere sowie
- Hetero-Aggregate, "Co-Aggregate", also Aggregate aus unterschiedlichen Monomeren.

Für das erfindungsgemäße Verfahren werden Proben aus dem menschlichen oder tierischen Körper verwendet und diesem nicht wieder zugeführt.

In einer Alternative des Verfahrens werden zur Ermittlung, insbesondere zur qualitativen Bestimmung von Krankheitsindikatoren Proben mit den potentiellen Biomarkern verglichen mit Proben gesund und/oder krank diagnostizierten Individuen. Diese Diagnose beruht z.B. auf einer Begutachtung der klinischen Präsentation oder Auswertung anderer Diagnoseverfahren. Durch den Vergleich der Menge der detektieren Aggregate ist eine Aussage über deren Eignung als Biomarker möglich.

In einer Alternative handelt es sich um Krankheitsindikatoren geeignet für den klinischen Routineeinsatz. Biomarker sind erfindungsgemäß für den klinischen Routineeinsatz geeignet sofern wissenschaftliche nachweisbare und reproduzierbare Beweise vorliegen, insbesondere auf Basis von physikalisch und/oder chemischen Methoden.

Als Substrat wird erfindungsgemäß ein Material gewählt, das eine möglichst geringe, unspezifische Bindungskapazität, insbesondere bezüglich der Aggregate fehlgefalteter Proteine besitzt.

In einer Ausführung der vorliegenden Erfindung wird ein Substrat aus Glas gewählt.

Das Substrat kann mit hydrophilen Stoffen, bevorzugt Poly-D-Lysin, Polyethylenglykol (PEG), bevorzugt heterobiofunktionales Polyethylenglykol (NHS-PEG-COOH) oder Dextran, insbesondere Dextran, bevorzugt Carboxymethyl-Dextran (CMD), beschichtet werden.

In einer Ausführung der vorliegenden Erfindung wird die Glasoberfläche hydroxyliert und anschließend mit Aminogruppen aktiviert.

Zur Vorbereitung des Substrats auf die Beschichtung können ein oder mehrere der folgenden Schritte durchgeführt werden:
- Waschen eines Substrats aus Glas bzw. eines Glasträgers im Ultraschallbad oder Plasma-cleaner, alternativ dazu in 5 M NaOH mindestens 3 Stunden inkubieren,
- Spülen mit Wasser und anschließendem Trocknen unter Stickstoff,
- Eintauchen in eine Lösung aus konzentrierter Schwefelsäure und Wasserstoffperoxid im Verhältnis 3:1 für die Aktivierung der Hydroxylgruppen,
- Spülen mit Wasser bis zu einer neutralen pH, anschließend mit Ethanol und Trocknen unter Stickstoffatmosphäre,
- Eintauchen in eine Lösung mit 3-Aminopropyltrietoxysilan (APTES) (1 - 7 %) in trockenem Toluol oder einer Lösung von Ethanolamin,
- Spülen mit Aceton oder DMSO und Wasser und Trocknen unter Stickstoffatmosphäre.

Für die Beschichtung mit Dextran, bevorzugt Carboxymethyl-Dextran (CMD), wird das Substrat mit einer wässrigen Lösung von CMD (in einer Konzentration von 10 mg/ml oder 20 mg/ml) und gegebenenfalls N-Ethyl-N-(3-Dimethylaminpropyl) Carbodiimid (EDC), (200 mM) und N-Hydroxysukzinimid (NHS), (50 mM) inkubiert und anschließend gewaschen.

Das Carboxymethyl-Dextran ist in einer Variante kovalent an die Glasoberfläche gebunden, die zunächst hydroxyliert und anschließend mit Amingruppen aktiviert wurde, wie oben beschrieben.

Als Substrat können auch Mikrotiterplatten, bevorzugt mit Glasboden, eingesetzt werden. Da bei der Verwendung von Polystryrolrahmen der Einsatz von konzentrierter Schwefelsäure nicht möglich ist, erfolgt die Aktivierung der Glasoberfläche in einer Ausführungsvariante der Erfindung analog Janissen et al. (Colloids Surf B Biointerfaces, 2009, 71(2), 200-207).

In einer Ausführung der vorliegenden Erfindung wird die Glasoberfläche mit Natronlauge (5 M) für 15 Minuten bei Raumtemperatur inkubiert, dreimal mit Wasser gespült, mit Salzsäure versetzt und erneut 15 Minuten bei Raumtemperatur inkubiert. Nach dreimaligem Waschen mit Wasser und zweimaligem Waschen mit Ethanol wird das Substrat (Glasoberfläche) unter Stickstoff-Atmosphäre getrocknet.

Zur Erzeugung von Aminogruppen auf der Glasoberfläche wird diese mit Ethanolamin (5,6 M) über Nacht bei Raumtemperatur inkubiert. Anschließend wird das Substrat dreimal mit DMSO, zweimal mit Ethanol gespült und unter Stickstoff-Atmosphäre getrocknet.

Heterobiofunktionales Polyethylenglykol (NHS-PEG-COOH, MW 3.400 Da) wird zu 50 mM in DMSO bei 70 °C für 1 min gelöst, auf Raumtemperatur abgekühlt und auf 2% Triethylamin eingestellt. Mit dieser Lösung wird mindestens eine Stunde bei Raumtemperatur inkubiert. Die Lösung wird aus den entfernt, und die Glasoberfläche dreimal mit Wasser gewaschen.

Zur Aktivierung der PEG-Beschichtung werden NHS und EDC zu jeweils 100 mM in MES-Puffer (0,1 M, pH 5) gelöst und im Verhältnis 1:1 zu Endkonzentrationen von jeweils 50 mM gemischt. Mit dieser Lösung wird das Substrat 30-60 Minuten inkubiert. Nach Entfernen der Lösung wird dreimal mit MES-Puffer (0,1 M, pH 5) gewaschen.

Der Fänger-Antikörper wird auf 30 ng/µl in PBS verdünnt und damit das Substart für 1-3 Stunden bei Raumtemperatur inkubiert. Anschließend wird die Lösung entfernt und dreimal mit PBST, dann dreimal mit PBS gewaschen.

3% BSA wurde zuvor bei 100.000 g (1 Stunde bei 4°C) zentrifugiert. Mit dem Überstand wird wird das Substart für eine Stunde bei Raumtemperatur inkubiert. Die BSA-Lösung wird entfernt und dreimal mit PBST gewaschen.

Die Probe (z.B. Aggregate aus rekombinantem Protein und natürliche Patientenprobe) wird - sofern erforderlich - in PBS verdünnt und aufgetragen. Das Substart mit Probe wird bei 1.000g für eine Stunde bei 4 °C in einem Ausschwingrotor zentrifugiert. Der Überstand wird entfernt und die Glasoberfläche dreimal mit PBST und dreimal mit PBS gewaschen.

Als Detektionssonden werden fluoreszenzmarkierte Antikörper eingesetzt. Diese werden auf 1-2 ng/µl in PBS verdünnt und mit 1.5% BSA versetzt. Die Ansätze werden bei 100.000 g für eine Stunde bei 4 °C zentrifugiert. Der Überstands wird auf das Substart aufgetragen und für 1-2 Stunden bei Raumtemperatur inkubiert. Anschließend wird die Lösung entfernt und fünfmal mit PBST und fünfmal mit PBS gewaschen.

Erfindungsgemäß werden auf dem Substrat Fängermoleküle immobilisiert, um die Aggregate fehlgefalteter, körpereigener Proteine zu fangen und fixieren.

Erfindungsgemäß werden als Fängermoleküle Antikörper der körpereigenen Proteine, die aggregieren, eingesetzt. Die Antikörper binden spezifisch ein Epitop der körpereigenen Proteine, die aggregieren.

In einer Alternative sind die Fängermoleküle kovalent an das Substrat gebunden.

In einer weiteren Alternative sind die Fängermoleküle kovalent mit der Beschichtung, bevorzugt Dextranschicht verbunden.

In einer Ausführung der vorliegenden Erfindung werden die Fängermoleküle (Antikörper), gegebenenfalls nach einer Aktivierung des mit CMD beschichteten Trägers durch eine Mischung aus EDC/NHS (200 bzw. 50 mM), auf dem Substrat immobilisiert.

Verbleibende Carboxylat-Endgruppen, an die keine Fängermoleküle gebunden wurden, können deaktiviert werden.

Zur Deaktivierung dieser Carboxylat-Endgruppen auf dem CMD-Spacer wird Ethanolamin in DMSO verwendet. Vor dem Auftrag der Proben werden die Substrate bzw. Träger mit PBS gespült.

Die zu vermessende Probe wird auf dem so präparierten Substrat inkubiert.

Erfindungsgemäß werden die Aggregate von Sonden markiert, die für eine spätere Detektion gekennzeichnet sind.

In einer Ausführung sind die Fängermoleküle und/oder die Sonde mit Fluoreszenzfarbstoffen gekennzeichnet.

In einer Alternative enthalten Fängermoleküle und/oder die Sonden spezifische Anti-Körper gegen ein Epitop der Proteine, die die Aggregate bilden.

In einer Variante der vorliegenden Erfindung werden als Sonden Antikörper eingesetzt. Fängermoleküle und Sonden können identisch sein.

In einer Ausführung der vorliegenden Erfindung unterscheiden sich Fängermoleküle und Sonden. So können z.B. unterschiedliche Antikörper als Fängermoleküle und Sonden eingesetzt werden.

Erfindungsgemäß werden mindestens 2 oder mehrere unterschiedliche Fängermoleküle und/ oder Sonden eingesetzt, die unterschiedliche Antikörper enthalten oder sind und gegebenenfalls auch unterschiedliche Farbstoffmarkierung haben.

Als Fängermoleküle können aber auch unterschiedliche Moleküle eingesetzt werden, wie z.B. unterschiedliche Antikörper oder Moleküle enthaltend Antikörper.

Ebenso können als Sonden mehrere, unterschiedliche Moleküle eingesetzt werden, wie z.B. unterschiedliche Antikörper enthalten oder sind.

Zur späteren Qualitätskontrolle der Oberfläche, zum Beispiel Gleichmäßigkeit der Beschichtung mit Fängermolekülen, können Fängermoleküle, gekennzeichnet mit Fluoreszensfarbstoffen, eingesetzt werden. Hierzu wird bevorzugt ein Farbstoff verwendet, der nicht mit der Detektion interferiert. Dadurch wird eine nachträgliche Kontrolle des Aufbaus möglich sowie eine Normierung der Messergebnisse.

Zur Detektion sind die Sonden so gekennzeichnet, dass sie ein optisch detektierbares Signal aussenden, ausgewählt aus der Gruppe bestehend aus Fluoreszenz-, Biolumineszenz- und Chemolumineszenz-Emission sowie Absorption.

In einer Alternative sind die Sonden mit Farbstoffen gekennzeichnet. Bevorzugt handelt es sich hierbei um Fluoreszenzfarbstoffe.

In einer Ausführung der vorliegenden Erfindung werden mindestens 2, 3, 4, 5, 6 oder mehr unterschiedliche Sonden eingesetzt. Die Sonden können sich sowohl bezüglich ihrer spezifischen Bindung an die Aggregate als auch bezüglich ihrer unterschiedlichen Kennzeichnung, z.B. durch Fluoreszenzfarbstoffe, unterscheiden.

Es können auch Sonden miteinander kombiniert werden, die geeignet sind FRET (Fluorescence Resonance Energy Transfer) als Detektion zu verwenden.

Der Einsatz mehrerer, unterschiedlicher Sonden, die durch unterschiedliche Fluroreszenzfarbstoffe gekennzeichnet sind, erhöht die Spezifität des bei der Messung erhaltenen Korrelationssignals. Zusätzlich wird dadurch auch das Ausblenden von Monomeren, also nicht aggregierten Molekülen möglich. Die Detektion von Monomeren kann insbesondere ausgeschlossen werden, falls Sonde und Fängermolekül identisch sind, oder beide ein überlappendes Epitop erkennen.

Als zu untersuchende Probe können körpereigene Flüssigkeiten oder Gewebe eingesetzt werden. In einer Ausführung der vorliegenden Erfindung wird die Probe ausgewählt aus Liquor (CSF), Blut, Plasma, Urin, Speichel, Schleimhaut und/oder Biopsie-Material . Die Proben können unterschiedliche, dem Fachmann bekannte, Aufbereitungsschritte durchlaufen.

Vorteil der vorliegenden Erfindung ist die Möglichkeit der Bestimmung von Aggregaten in unbehandelten Proben, bevorzugt CSF.

In einer Variante der vorliegenden Erfindung werden interne oder externe Standards eingesetzt.

Solche Standards enthalten oder sind ein Polymer, aufgebaut aus Monomer-Sequenzen, mindestens gemäß SEQ ID NO: 10 und/oder SEQ ID NO: 11, wobei diese Polymere nicht aggregieren und es sich bei den körpereigenen Proteinen um jene handelt, die aggregieren.

Erfindungsgemäß wird eine Monomer-Detektion von körpereigenen Proteinen ausgeschlossen indem im Testsystem drei unterschiedliche bzw. drei unterschiedlich markierte Sonde eingesetzt werden, die an einem ähnlichen bzw. gleichen Epitop binden. Alternativ oder zusätzlich kann die Detektion von Monomeren ausgeschlossen werden indem Signale mit einer niedrigeren Intensität durch ein Intensität-cut-off nicht gewertet werden. Da größere Aggregate mehrere Bindungsstellen für die beiden mit unterschiedlichen markierten Farbstoffen Sonde besitzen, kann eine Monomer-Detektion alternativ oder zusätzlich durch Kreuzkorelation dieser Signale ausgeschlossen werden.

Die Detektion der markierten Aggregate erfolgt durch Scannen oder andere Arten der Oberflächenabbildung. Die Detektion erfolgt bevorzugt mit konfokaler Fluoreszenzmikroskopie, Fluoreszenzkorrelationsspektroskopie (FCS), insbesondere in Kombination mit Kreuzkorrelation und Single-Particle-Immunosolvent Laserscanning-Assay und/oder Laser-Scanning-Mikroskop (LSM). Alternativ erfolgt die Detektion mittels Total Internal Reflection Fluoreszenz Mikroskopie (TIRFM).

Die Detektion wird in einer Alternative der vorliegenden Erfindung mit einem konfokalen Laser-Scanning-Mikroskop durchgeführt.

In einer Ausführung der vorliegenden Erfindung wird ein Laserfokus, wie er z.B. in der Laser-Scanning-Mikroskopie verwendet wird, oder ein FCS (Fluorescence Correlation Spectroscopy System), dazu eingesetzt, sowie die entsprechenden superauflösenden Varianten wie zum Beispiel STED oder SIM. Alternativ dazu kann die Detektion durch ein TIRF-Mikroskop erfolgen, sowie die entsprechenden superauflösenden Varianten davon, wie zum Beispiel STORM, dSTORM.

Demgemäß sind in der Ausführung der Erfindung Verfahren, die auf einem nichtortsaufgelöstem Signal beruhen, wie ELISA oder Sandwich-ELISA, ausgeschlossen.

Bei der Detektion ist eine hohe Ortsauflösung vorteilhaft. In einer Ausführung des erfindungsgemäßen Verfahrens werden dabei so viele Datenpunkte gesammelt, dass die Detektion eines Aggregates vor einem Hintergrundsignal, welches z.B. durch gerätespezifisches Rauschen, andere unspezifische Signale oder unspezifisch gebundene Sonden verursacht wird, ermöglicht. Auf diese Weise werden so viele Werte ausgelesen (Readout-Werte), wie orts-aufgelöste Ereignisse, wie z.B. Pixel, vorhanden sind. Durch die Ortsauflösung wird jedes Ereignis vor dem jeweiligen Hintergrund bestimmt und stellt so einen Vorteil gegenüber ELISA-Verfahren mit nur einem Readout-Wert, wenigen Readout-Werten und/oder ohne ortsaufgelöstem Signal dar.

In einer Alternative werden in dem erfindungsgemäßen Verfahren mehrere unterschiedliche Sonden eingesetzt. Dadurch wird die Information, d.h. die ausgelesenen Werte vervielfacht, da für jeden Punkt, für jedes Aggregat oder für jedes Detektionsereignis eine separate Information erhalten wird in Abhängigkeit der jeweiligen Sonde, die das Signal liefert. So wird für jedes Ereignis die Spezifität des Signals erhöht. Dadurch kann für jedes detektierte Aggregat auch seine Zusammensetzung bestimmt werden, d.h. die Art des Aggregats, also die Zusammensetzung aus Monomeren oder Mischungen daraus.

Die Zahl der unterschiedlichen Sonden wird dabei nur durch die Interferenz der zu verwendenden Fluoreszenzfarbstoffen begrenzt. So können 2, 3, 4 oder mehrere unterschiedliche Sonden-Farbstoff-Kombinationen verwendet werden.

Wesentlich für die Auswertung gemäß dem oben beschriebenen Verfahren, wenn mehr als eine Sonde verwendet wird, sind orts- und zeitaufgelöste Informationen. Dabei kann es sich z.B. um die Art und/oder Intensität der Fluoreszenz handeln. Bei Auswertung dieser Daten für alle eingesetzten und detektierten Sonden wird erfindungsgemäß die Anzahl der Aggregate, deren Form, Größe und/oder deren Zusammensetzung bestimmt. Dabei können Informationen über die Größe der Oligomere direkt oder indirekt erhalten werden, abhängig davon, ob die Partikel kleiner oder größer als die Zeit- oder Ortsauflösung der verwendeten Abbildungsverfahren sind, in einer Ausführung können Algorithmen zur Hintergrundminimierung eingesetzt werden und/oder Intensität-Schwellenwerte angewendet werden.

Als Fluoreszenzfarbstoff können die dem Fachmann bekannten Farbstoffe eingesetzt werden. Alternativ können GFP (Green Fluorescence Protein), Konjugate und/oder Fusionsproteine davon, sowie Quantendots verwendet werden.

Durch die Verwendung von internen oder externen Standards sind Testergebnisse objektiv miteinander vergleichbar und damit aussagekräftig.

In einer Ausführung der vorliegenden Erfindung werden ein interner oder externer Standard zur Quantifizierung von Aggregaten eingesetzt.

In Anlehnung an die Analyse der Verteilung der Fluoreszenzintensität (FIDA-Fluoreszenz-Intensity-Distribution-Analysis) handelt es sich bei dem erfindungsgemäßen Verfahren um ein sog. Oberflächen-FIDA (Surface-FIDA, sFIDA). Die Detektion erfolgt mit konfokaler Fluoreszenzmikroskopie, Fluoreszenzkorrelationsspektroskopie (FCS), Laser-Scanning-Mikroskop (LSM) und/oder Total Internal Reflection Fluoreszenz Mikroskopie (TIRFM), bevorzugt LSM und/oder Total Internal Reflection Fluoreszenz Mikroskopie (TIRFM).

In einer Ausführung der Erfindung werden pro Well bis zu 50 Einzelbilder (1000x1000 Pixel, 114 nm/pixel) pro Fluoreszenzkanal mit einer hochsensitiven CCD-Kamera abgebildet.

Hintergrundsignale in den Bilddaten werden über einen Intensitätsschwellenwert bereinigt. Die mittlere Anzahl der Pixel mit Graustufenwerten oberhalb des Schwellenwerts wird bestimmt (sFIDA-readout). Kommen mehrere Detektionssonden zum Einsatz, werden ausschließlich die in allen Fluoreszenzkanälen colokalisierten Ereignisse gewertet.

Das sFIDA-Verfahren wurde in Bannach et al. (2012. Detection of prion protein particles in blood plasma of scrapie infected sheep. PloS one 7, e36620) sowie Wang-Dietrichet al. (2013. The amyloid-beta oligomer count in cerebrospinal fluid is a biomarker for Alzheimer's disease. Journal of Alzheimer's disease 34, 985-994) beschrieben, die unter Bezugnahme eingeschlossen sind.

Durch Wahl der Fänger - und Sonden-Moleküle lässt sich bestimmen, welche Größe die Oligomere besitzen müssen, um zur Detektion (Signal) beitragen zu können. So können zum Beispiel Monomere, Dimere, Trimere etc. detektiert werden. Es können aber auch Fänger - und Sonden-Moleküle eingesetzt werden, dass als kleinste detektierbare Einheit zum Beispiel Dimere oder andere Polymere sind.

Mit dem erfindungsgemäßen Verfahren ist zusätzlich auch die genaue Analyse von kleinen, frei diffundierbaren Aggregate möglich. Aufgrund ihrer Größe, die unterhalb ihrer Auflösung für Lichtmikroskope liegt, konnten kleine Oligomere schwer von der Hintergrundfluoreszenz (verursacht z.B. durch nicht gebundene Antikörper) unterschieden werden.

Neben der extrem hohen Sensitivität zeigt das erfindungsgemäße Verfahren auch eine Linearität in Bezug auf die Anzahl der Aggregate über einen großen Bereich.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung: :
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht überzogen). Darauf werden (bevorzugt kovalent) Fängermoleküle für AA-enthaltende Partikel (z.B. anti-AA-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene AA-Partikel (z.B. AA-Aggregate) werden mit einer für die weitere Detektion dienlichen Sonde (z.B. einem Fluoreszenzfarbstoff-gebundene anti-AA-Antikörper) markiert. Bevorzugte Fluoreszenzfarbstoffe könnten u.a. auch Quantumdots sein. Bevorzugt werden die AA-enthaltende Partikel mit mindestens einer weiteren Sonde (z.B. einem Fluoreszenzfarbstoff-gebunden anti-AA-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AA-Monomeren. Besonders, wenn eine der Detektionssonden identisch mit dem Fängermolekül (aus Schritt 1) ist oder beide ein überlappendes Epitop erkennen, kann die Detektion von AA-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde AA-Antikörper, bevorzugt Anti-Serum Amyloid A antibody clone 115, mc1 und/oder 291 eingesetzt.In einer Alternative werden als Fängermolekül und/oder Sonde (AF488-markiert): EPR 4134 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird oder zum Beispiel TIRFM) oder durch andere Arten der Oberflächen-Abbildung (z.B. durch ein TIRF-Mikroskop). Je höher die Zeit und- Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für AL-enthaltende Partikel (z.B. anti-AL-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene AL-Partikel (z.B. AL-Aggregate) werden mit einer für die weitere Detektion dienlichen Sonde (z.B. einem Fluoreszenzfarbstoff-gebundene anti-AL-Antikörper) markiert. Bevorzugte Fluoreszenzfarbstoffe könnten u.a. auch Quantumdots sein. Bevorzugt werden die AL-enthaltende Partikel mit mindestens einer weiteren Sonde (z.B. einem Fluoreszenzfarbstoff-gebunden anti-AL-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AL-Monomeren. Besonders, wenn eine der Detektionssonden identisch mit dem Fängermolekül (aus Schritt 1) ist oder beide ein überlappendes Epitop erkennen, kann die Detektion von AL-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde AAnti-Lambda Light chain antibody clone EPR5367, HP6054 und/oder 2G9 eingesetzt. In einer Alternative werden als Fängermolekül und/oder Sonde (AF488-markiert): EPR 5367 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für AApoAI-enthaltende Partikel (z.B. anti-AApoAI-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene AApoAI-Partikel (z.B. AApoAI-Aggregate) werden mit einer für die weitere Detektion dienlichen Sonde (z.B. einem Fluoreszenzfarbstoff-gebundene anti-AApoAI-Antikörper) markiert. Bevorzugte Fluoreszenzfarbstoffe könnten u.a. auch Quantumdots sein. Bevorzugt werden die AApoAI-enthaltende Partikel mit mindestens einer weiteren Sonde (z.B. einem Fluoreszenzfarbstoff-gebunden anti-AApoAI-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AApoAI-Monomeren. Besonders, wenn eine der Detektionssonden identisch mit dem Fängermolekül (aus Schritt 1) ist oder beide ein überlappendes Epitop erkennen, kann die Detektion von AApoAI-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Apolipoprotein Al antibody clone 12C8, 1409 und/oder G2 eingesetzt. In einer Alternative werden als Fängermolekül und/oder Sonde (AF488-markiert): EPR 1368Y eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für AApoAII-enthaltende Partikel (z.B. anti-AApoAll-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe a enthaltene AApoAII-Partikel (z.B. AApoAII-Aggregate) werden mit einer für die weitere Detektion dienlichen Sonde (z.B. einem Fluoreszenzfarbstoff-gebundene anti-AApoAll-Antikörper) markiert. Bevorzugte Fluoreszenzfarbstoffe könnten u.a. auch Quantumdots sein. Bevorzugt werden die AApoAII-enthaltende Partikel mit mindestens einer weiteren Sonde (z.B. einem Fluoreszenzfarbstoff-gebunden anti-AApoAll-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von AApoAII-Monomeren. Besonders, wenn eine der Detektionssonden identisch mit dem Fängermolekül (aus Schritt 1) ist oder beide ein überlappendes Epitop erkennen, kann die Detektion von AApoAII-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Apolipoprotein AII antibody clone 4F3, EPR2913 und/oder EP2912 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für ATTR-enthaltende Partikel (z.B. anti-ATTR-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltende ATTR-Partikel (z.B. ATTR-Aggregate) werden mit einer für die weitere Detektion dienlichen Sonde (z.B. einem Fluoreszenzfarbstoff-gebundene anti-ATTR-Antikörper) markiert. Bevorzugte Fluoreszenzfarbstoffe könnten u.a. auch Quantumdots sein. Bevorzugt werden die ATTR-enthaltende Partikel mit mindestens einer weiteren Sonde (z.B. einem Fluoreszenzfarbstoff-gebunden anti-ATTR-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von ATTR-Monomeren. Besonders, wenn eine der Detektionssonden identisch mit dem Fängermolekül (aus Schritt 1) ist oder beide ein überlappendes Epitop erkennen, kann die Detektion von ATTR-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Prealbumin antibody clone EP2929Y, EPR3119 und/oder 10E1 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für DISC1-enthaltende Partikel (z.B. anti-DISC1-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene DISC1-Partikel (z.B. DISC1-Aggregate) werden mit einer für die weitere Detektion dienlichen Sonde (z.B. einem Fluoreszenzfarbstoff-gebundene anti-DISC1-Antikörper) markiert. Bevorzugte Fluoreszenzfarbstoffe könnten u.a. auch Quantumdots sein. Bevorzugt werden die DISC1-enthaltende Partikel mit mindestens einer weiteren Sonde (z.B. einem Fluoreszenzfarbstoff-gebunden anti-DISC1-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von DISC1-Monomeren. Besonders, wenn eine der Detektionssonden identisch mit dem Fängermolekül (aus Schritt 1) ist oder beide ein überlappendes Epitop erkennen, kann die Detektion von DISC1-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-DISC1 antibody clone 14F2, 2C7 und/oder FFD5 eingesetzt. In einer Alternative werden Anti-DISC1-AK 14F2 als Fänger-Antikörper und 14F2-AF633 als Detektionssonde eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für FUS-enthaltende Partikel (z.B. anti-FUS-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene FUS-Partikel (z.B. FUS-Aggregate) werden mit einer für die weitere Detektion dienlichen Sonde (z.B. einem Fluoreszenzfarbstoff-gebundene anti-FUS-Antikörper) markiert. Bevorzugte Fluoreszenzfarbstoffe könnten u.a. auch Quantumdots sein. Bevorzugt werden die FUS-enthaltende Partikel mit mindestens einer weiteren Sonde (z.B. einem Fluoreszenzfarbstoff-gebunden anti-FUS-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von FUS-Monomeren. Besonders, wenn eine der Detektionssonden identisch mit dem Fängermolekül (aus Schritt 1) ist oder beide ein überlappendes Epitop erkennen, kann die Detektion von FUS-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-TLS/FUS antibody clone EPR5812, EPR5813 und/oder CL0190 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für IAPP-enthaltende Partikel (z.B. anti-IAPP-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene IAPP-Partikel (z.B. IAPP-Aggregate) werden mit einer für die weitere Detektion dienlichen Sonde (z.B. einem Fluoreszenzfarbstoff-gebundene anti-IAPP-Antikörper) markiert. Bevorzugte Fluoreszenzfarbstoffe könnten u.a. auch Quantumdots sein. Bevorzugt werden die IAPP-enthaltende Partikel mit mindestens einer weiteren Sonde (z.B. einem Fluoreszenzfarbstoff-gebunden anti-IAPP-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von IAPP-Monomeren. Besonders, wenn eine der Detektionssonden identisch mit dem Fängermolekül (aus Schritt 1) ist oder beide ein überlappendes Epitop erkennen, kann die Detektion von IAPP-Monomeren ausgeschlossen werden. Zur Steigerung der Spezifität kann als zusätzliche Detektionssonde der amyloidspezifische Farbstoff Thioflavin T eingesetzt werden.
   In einer Alternative wird als Fängermolekül und/oder Sonde Anti-Amylin antibody clone R10/99 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für SOD1-enthaltende Partikel (z.B. anti-SOD1-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene SOD1-Partikel (z.B. SOD1-Aggregate) werden mit einer für die weitere Detektion dienlichen Sonde (z.B. einem Fluoreszenzfarbstoff-gebundene anti-SOD1-Antikörper) markiert. Bevorzugte Fluoreszenzfarbstoffe könnten u.a. auch Quantumdots sein. Bevorzugt werden die SOD1-enthaltende Partikel mit mindestens einer weiteren Sonde (z.B. einem Fluoreszenzfarbstoff-gebunden anti-SOD1-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von SOD1-Monomeren. Besonders, wenn eine der Detektionssonden identisch mit dem Fängermolekül (aus Schritt 1) ist oder beide ein überlappendes Epitop erkennen, kann die Detektion von SOD1-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Superoxide Dismutase 1 antibody clone 2F5, 71G8 und/oder EPR1726 eingesetzt. In einer Alternative werden als Fängermolekül und/oder Sonde (AF488-markiert): EPR 1726 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assays, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für α-Synuclein-enthaltende Partikel (z.B. anti-α-Synuclein-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene α-Synuclein-Partikel (z.B. α-Synuclein-Aggregate) werden mit einer für die weitere Detektion dienlichen Sonde (z.B. einem Fluoreszenzfarbstoff-gebundene anti-α-Synuclein-Antikörper) markiert. Bevorzugte Fluoreszenzfarbstoffe könnten u.a. auch Quantumdots sein. Bevorzugt werden die α-Synuclein-enthaltende Partikel mit mindestens einer weiteren Sonde (z.B. einem Fluoreszenzfarbstoff-gebunden anti-α-Synuclein-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von α-Synuclein-Monomeren. Besonders, wenn eine der Detektionssonden identisch mit dem Fängermolekül (aus Schritt 1) ist oder beide ein überlappendes Epitop erkennen, kann die Detektion von α-Synuclein-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Alpha-Synuclein antibody clone 2B2A11, 3H2897 und/oder 211 eingesetzt. In einer Alternative werden als Fänger-Antikörper Anti-aSyn-AB 2B2A11 und als Detektionssonde die fluoreszenzmarkierten Anti-aSyn-ABs 3H2897-AF633 bzw. 211-AF488 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

In einer Ausführung wird die Aufgabe durch folgendes Verfahren, sogenannter sFIDA-Assay, gelöst:
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für Tau-enthaltende Partikel (z.B. anti-Tau-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene Tau-Partikel (z.B. Tau-Aggregate) werden mit einer für die weitere Detektion dienlichen Sonde (z.B. einem Fluoreszenzfarbstoff-gebundene anti-Tau-Antikörper) markiert. Bevorzugte Fluoreszenzfarbstoffe könnten u.a. auch Quantumdots sein. Bevorzugt werden die Tau-enthaltende Partikel mit mindestens einer weiteren Sonde (z.B. einem Fluoreszenzfarbstoff-gebunden anti-Tau-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von Tau-Monomeren. Besonders, wenn eine der Detektionssonden identisch mit dem Fängermolekül (aus Schritt 1) ist oder beide ein überlappendes Epitop erkennen, kann die Detektion von Tau-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Tau antibody clone E178, Tau46 und/oder Tau 5 eingesetzt. In einer Alternative werden als Fängermolekül und/oder Sonde Anti-Tau 6E10/Atto488 und/oder Nab228 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für TDP-43-enthaltende Partikel (z.B. anti-TDP-43-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene TDP-43-Partikel (z.B. TDP-43-Aggregate) werden mit einer für die weitere Detektion dienlichen Sonde (z.B. einem Fluoreszenzfarbstoff-gebundene anti-TDP-43-Antikörper) markiert. Bevorzugte Fluoreszenzfarbstoffe könnten u.a. auch Quantumdots sein. Bevorzugt werden die TDP-43-enthaltende Partikel mit mindestens einer weiteren Sonde (z.B. einem Fluoreszenzfarbstoff-gebunden anti-TDP-43-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von TDP-43-Monomeren. Besonders, wenn eine der Detektionssonden identisch mit dem Fängermolekül (aus Schritt 1) ist oder beide ein überlappendes Epitop erkennen, kann die Detektion von TDP-43-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-TARDBP antibody clone EPR5810, 3H8 und/oder K1B8 eingesetzt.In einer Alternative werden als Fängermolekül und/oder Sonde (AF488-markiert): EPR 5810 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für Huntingtin-enthaltende Partikel (z.B. anti-Huntingtin-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltende Huntingtin-Partikel (z.B. Huntingtin-Aggregate) werden mit einer für die weitere Detektion dienlichen Sonde (z.B. einem Fluoreszenzfarbstoff-gebundene anti-Huntingtin-Antikörper) markiert. Bevorzugte Fluoreszenzfarbstoffe könnten u.a. auch Quantumdots sein. Bevorzugt werden die Huntingtin-enthaltende Partikel mit mindestens einer weiteren Sonde (z.B. einem Fluoreszenzfarbstoff-gebunden anti-Huntingtin-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von Huntingtin-Monomeren. Besonders, wenn eine der Detektionssonden identisch mit dem Fängermolekül (aus Schritt 1) ist oder beide ein überlappendes Epitop erkennen, kann die Detektion von Huntingtin-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Huntingtin antibody clone EP867Y, D7F7 und/oder HIP-1 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Im Folgenden wird ein Verfahren zur qualitativen und/oder quantitativen Bestimmung von Krankheitsindikatoren beschrieben. Dieses ist nicht erfindungsgemäß und dient nur der Veranschaulichung::
1. Eine Oberfläche (bevorzugt aus Glas) wird vorbehandelt, so dass sie eine möglichst geringe unspezifische Bindungskapazität besitzt (z.B. durch chemische Modifikation kovalent mit einer Dextran-Schicht oder Polyethylenglykol überzogen). Darauf werden (bevorzugt kovalent) Fängermoleküle für ALys-enthaltende Partikel (z.B. anti-ALys-Antikörper) gebunden. Die Fängermoleküle können alle identisch sein oder Mischungen verschiedener Fängermoleküle sein.
2. Auf die so vorbereitete Oberfläche wird dann die zu untersuchende Probe (z.B. Liquor, Blut, Plasma, Urin, Speichel, Schleimhaut, Biopsie-Material) mit oder ohne vorherige Aufbereitungsschritte aufgetragen. Unspezifisch gebundene Substanzen können durch Waschschritte entfernt werden.
3. Potentiell in der Probe enthaltene ALys-Partikel (z.B. ALys-Aggregate) werden mit einer für die weitere Detektion dienlichen Sonde (z.B. einem Fluoreszenzfarbstoff-gebundene anti-ALys-Antikörper) markiert. Bevorzugte Fluoreszenzfarbstoffe könnten u.a. auch Quantumdots sein. Bevorzugt werden die ALys-enthaltende Partikel mit mindestens einer weiteren Sonde (z.B. einem Fluoreszenzfarbstoff-gebunden anti-ALys-Antikörper) markiert. Der Einsatz mehrerer unterschiedlicher Sonden, die an unterschiedliche Fluoreszenzfarbstoffen gekoppelt sind, erhöht zum einen die Spezifität des am Ende erhaltenen Korrelationssignals, zum anderen ermöglicht dies auch das Ausblenden von ALys-Monomeren. Besonders, wenn eine der Detektionssonden identisch mit dem Fängermolekül (aus Schritt 1) ist oder beide ein überlappendes Epitop erkennen, kann die Detektion von ALys-Monomeren ausgeschlossen werden. Zur Steigerung der Spezifität kann als zusätzliche Detektionssonde der amyloidspezifische Farbstoff Thioflavin T eingesetzt werden.
   In einer Alternative werden als Fängermolekül und/oder Sonde Anti-Lysozyme antibody clone BGN/06/961 und/oder BGN/0696/2B10 eingesetzt. In einer Alternative werden als Fängermolekül und/oder Sonde (AF488-markiert): EPR 2994 eingesetzt.
4. Die Schritte 2 und 3 können auch vertauscht werden. Durch geeignete Waschschritte werden überschüssige Sonden entfernt.
5. Die Detektion der markierten Aggregate erfolgt durch Scannen (z.B. durch einen Laserfocus, wie er z.B. in der Laser Scanning Mikroskopie verwendet wird) oder durch andere Arten der Oberflächen-Abbildung (z.B. durch ein TIRF-Mikroskop). Je höher die Zeit- und Ortsauflösung dabei ist, desto mehr Datenpunkte entstehen, die jeweils die Detektion eines Aggregates vor einem Hintergrundsignal (z.B. durch gerätespezifisches Rauschen, unspezifische
   Signale, unspezifisch gebunden Sonden) erlauben. Auf diese Art entsteht nicht nur ein Readout-Wert (wie dies bei einem ELISA der Fall wäre), sondern so viele Readout-Werte, wie Zeit- und Orts-aufgelöste Ereignisse (z.B. Pixel) vorhanden sind. Durch den Einsatz mehrerer unterschiedlicher Sonden (siehe Schritt 3) wird diese Information sogar vervielfacht und für jeden Punkt, für jedes Aggregat oder für jedes Detektionsereignis kann die Information separat erhalten werden, welche der Sonden dort Signale liefert. So kann für jedes Ereignis die Spezifität des Signales erhöht werden.
6. Zur Auswertung werden die Zeit- und Orts-aufgelösten Informationen (z.B. die Fluoreszenz-Intensität) aller eingesetzten und detektierten Sonden herangezogen, um z.B. die Anzahl der Aggregate, deren Größe und deren Zusammensetzung zu bestimmen. Dabei können z.B. auch Algorithmen der Hintergrundminimierung eingesetzt werden und/oder auch Intensitäts-Schwellenwerte für die weitere Auswertung angewandt werden.
7. Um die Testergebnisse miteinander (über Entfernungen, Zeiten und Experimentatoren hinweg) vergleichbar zu machen, können Standards (interne und/oder externe) eingesetzt werden.

Erfindungsgemäß ist der Gegenstand der vorliegenden Erfindung ein Verfahren zur selektiven Quantifizierung und/oder Charakterisierung von Aggregaten des Typs α-Synuclein-Aggregate und Tau-Aggregate.

Erfindungsgemäß ist ein Verfahren zur selektiven Quantifizierung von Aggregaten des Typs α-Synuclein-Aggregaten in Körperflüssigkeiten als Biomarker für die Parkinson-Krankheit und anderer Synucleinopathien, wobei zusätzlich auf einen weitere Aggregat-Typ untersucht wird, nämlich auf Tau-Aggregate.

Erfindungsgemäß ist der Gegenstand der vorliegenden Erfindung ein Verfahren zur selektiven Quantifizierung und/oder Charakterisierung von gemischten Aggregaten des Typs α-Synuclein-Aggregate mit Tau-Aggregate in Körperflüssigkeiten als Biomarker für Parkinson-Krankheit und anderer Synucleinopathien.

Erfindungsgemäß ist ein Verfahren zur selektiven Quantifizierung und/oder Charakterisierung von Aggregaten des Typs Tau-Aggregaten in Körperflüssigkeiten als Biomarker für Tauopathien, wobei zusätzlich auf einen weitere Aggregat-Typ untersucht wird, nämlich auf α-Synuclein-Aggregate, .

Erfindungsgemäß ist der Gegenstand der vorliegenden Erfindung ein Verfahren zur selektiven Quantifizierung und/oder Charakterisierung von gemischten Aggregaten des Typs Tau-Aggregate mit α-Synuclein-Aggregate in Körperflüssigkeiten als Biomarker für Tauopathien.

Erfindungsgemäß ist ein Verfahren zur selektiven Quantifizierung und/oder Charakterisierung von α-Synuclein-Aggregate und Tau-Aggregaten, in Körperflüssigkeiten als Biomarker für Alzheimer-Demenz.

Gegenstand der vorliegenden Erfindung ist ferner die Verwendung eines Standards zur selektiven Quantifizierung und/oder Charakterisierung der Krankheitsindikatoren in einem erfindungsgemäßen Verfahren, wobeies sich bei dem Standard um ein Dendrimer handelt, welches ein zentrales Gerüstmolekül und Epitope der Proteine α-Synuclein-Aggregate oder Tau-Aggregate gemäß SEQ ID NO: 10 oder SEQ ID NO: 11 enthält, wobei sowohl Standards, die aus den gleichen Monomer-Sequenzen aufgebaut sind, als auch solche, die aus unterschiedlichen Monomer-Sequenzen aufgebaut sind verwendet werden.

Als Standard im Sinne der vorliegenden Erfindung wird eine allgemein gültige und akzeptierte, feststehende Bezugsgröße bezeichnet, die zum Vergleichen und Bestimmen von Eigenschaften und/oder Menge dient, insbesondere zur Bestimmung der Größe und Menge von (pathogenen) Aggregaten aus körpereigenen (fehlgefalteten) Proteinen. Der Standard im Sinne der vorliegenden Erfindung kann zum Kalibrieren von Geräten und/oder Messungen verwendet werden.

Wesentlich für die Standards ist, dass die Standards nicht aggregieren, bevorzugt durch die Verwendung von Eptitop-Sequenzen, die nicht aggregieren, da der entsprechende Teilbereich körpereigener Proteine für die Aggregation nicht verantwortlich ist, oder die durch Blockierung der für die Aggregation verantwortlichen Gruppen nicht aggregieren.

In diesem Sinne beschreibt hier der Begriff "Epitop-Sequenz" einen Teilbereich, ein Fragment der einzelnen Proteine (Monomeren) die Aggregate fehlgefalteter Proteine bilden.

In einer Ausführung ist der Teilbereich ein Epitop gemäß einer der SEQ ID NO: 10 oder 11.

Eine so ausgewählte Epitop-Sequenz wird in der gewünschten Anzahl bei dem Aufbau der erfindungsgemäßen Standards eingebaut und/oder miteinander erfindungsgemäß verknüpft.

Die erfindungsgemäßen Standards sind Polymere, die aus den oben beschriebenen Epitopen aufgebaut sind, gegebenenfalls enthaltend weitere Elemente.

Das erfindungsgemäße Standardmolekül ist ein Polymer aus den oben definierten Epitopen. Oligomer im Sinne der Erfindung ist ein Polymer gebildet aus 2, 3, 4, 5, 6, 7, 8, 9, 10, 11, 12, 13, 14, 15, 16, 17, 18, 19 oder 20 Monomer-Sequenz, oder Vielfachen davon, bevorzugt 2 - 16, 4-16, 8-16, besonders bevorzugt 8 oder 16, oder Vielfachen davon.

In einer Alternative der vorliegenden Erfindung sind die Standards wasserlöslich.

In einer Alternative der vorliegenden Erfindung sind die erfindungsgemäßen Standard aus gleichen Epitop-Sequenz aufgebaut.

In einer Alternative der vorliegenden Erfindung sind die erfindungsgemäßen Standard aus unterschiedlichen Epitop-Sequenz aufgebaut.

Wesentlich für die erfindungsgemäß eingesetzten Sequenzen ist ihre Eigenschaft nicht (oder nur gemäß den Bedingungen kontrolliert) zu aggregieren und/oder ihre die Aktivität als Epitop.

Standards haben in einer Alternative vorteilhaft eine höhere Löslichkeit im Wässrigen als pathogene Aggregate oder Oligomere aus körpereigenen Proteinen.

In einer Ausführung der vorliegenden Erfindung besitzen die Standards eine genau definierte Anzahl von Epitopen, die kovalent miteinander verknüpft sind (unmittelbar oder über Aminosäuren, Spacer und/oder funktionelle Gruppen) für die Bindung der entsprechenden Bindungspartner.

In diesem Sinne mit Bezug auf die Standards werden Bindungspartner ausgewählt aus der Gruppe bestehend aus: Antikörper, Nanobody und Affibody. Bindungspartner sind darüber hinaus alle Moleküle, die eine hinreichende Bindespezifität für das zu detektierende Aggregat besitzen, z.B. Farbstoffe (ThioflavinT, Kongorot, etc.).

Ein erfindungsgemäßes Standardmolekül kann Epitope für mindestens 2, 3, 4, 5, 6, 7, 8, 9, 10 oder mehr verschiedene Bindungspartner enthalten.

Epitope charakteristisch für verschiedene Bindungspartner können in die erfindungsgemäßen Standards dadurch eingebaut werden, dass Monomer-Sequenzen verwendet werden, die identisch mit unterschiedlichen Bereichen eines oder unterschiedlicher Proteine (die Aggregate fehlgefalteter Proteine bilden) sind, bzw. mindestens eine 50 %ige Homologie dazu aufweisen, aber die Aktivität des entsprechenden Epitops besitzen.

In einer Ausführung der vorliegenden Erfindung enthalten die Standardmoleküle sog. Spacer.

Unter einem Spacer ist ein Molekül zu verstehen, das über kovalente Bindungen in das Standardmolekül eingebaut ist, und bestimmte physikalische und/oder chemische Eigenschaften besitzt, durch welche die Eigenschaften des Standardmoleküls verändert werden. In einer Ausführung der erfindungsgemäßen Standards werden hydrophile oder hydrophobe, bevorzugt hydrophile Spacer, eingesetzt. Hydrophile Spacer werden ausgewählt aus der Gruppe der Moleküle, gebildet aus Polyethylenglycol, Zucker, Glycerin, Poly-L-Lysin oder beta-Alanin.

Die erfindungsgemäßen Standards enthalten in einer Alternative der vorliegenden Erfindung (weitere) funktionelle Gruppen.

Unter funktionellen Gruppen sind Moleküle zu verstehen, die kovalent an die Standardmoleküle gebunden sind. In einer Variante enthalten die funktionellen Gruppen Biotin-Gruppen. Dadurch wird eine starke kovalente Bindung an Streptavidin ermöglicht. Standardmoleküle enthaltend Biotin-Gruppen können so an Moleküle, enthaltend Streptavidin-Gruppen, gebunden werden. Falls die erfindungsgemäßen Standardmoleküle Biotin und/oder Streptavidin-Gruppen enthalten, können so größere Standards zusammengebaut werden oder mehrere, ggf. unterschiedliche Standardmoleküle, an ein Gerüst gebunden werden.

In einer weiteren Alternative der vorliegenden Erfindung enthalten die Standardmoleküle Farbstoffe zur spektralphotometrischen Bestimmung und/oder aromatische Aminosäuren. Aromatische Aminosäuren sind z.B. Tryptophane, Tyrosin, Phenylalanin oder Histidin, bzw. ausgewählt aus dieser Gruppe. Durch den Einbau von Tryptophan wird eine spektralphotometische Bestimmung der Konzentration von Standards in Lösung ermöglicht.

Erfindungsgemäß sind die Standards als Dendrimere aufgebaut. Die erfindungsgemäßen Dendrimere sind aus den oben beschriebenen erfindungsgemäß zu verwendenden Monomer-Sequenzen aufgebaut und können ein zentrales Gerüstmolekül enthalten. Bevorzugt ist das Gerüstmolekül ein Streptavidin-Monomer, besonders bevorzugt ein Polymer, insbesondere Tetramer.

Die erfindungsgemäßen Dendrimere können jede der oben beschriebenen Merkmale der Standards oder jede beliebige Kombination davon enthalten.

In einer Alternative der vorliegenden Erfindung handelt es sich um:
- Dendrimere enthaltend eine genau definierte Anzahl von Epitopen für die kovalente Bindung von Bindungspartnern,
- Dendrimer dadurch gekennzeichnet, dass er eine höhere Löslichkeit im Wässrigen besitzt, als die pathogenen Aggregate aus körpereigenen Proteinen,
- Dendrimer enthaltend funktionelle Gruppen,
- Dendrimer enthaltend mindestens ein Spacer-Molekül und/oder
- Dendrimer enthaltend Farbstoffe zur spektralphotometrischen Bestimmung und/oder aromatische Aminosäuren.

Erfindungsgemäß haben die Dendrimere eine radiale Symmetrie.

In einer Variante erfolgt die Verzweigung der ersten Generation des Dendrimers über Lysin, inbesondere drei Lysin-Aminosäuren.

In einer weiteren Alternative der vorliegenden Erfindung sind in den Standards, insbesondere Dendrimeren, die Epitope, nicht über eine Bindung zu einem Schwefelatom, nicht über eine Thioether-Bindung und/oder nicht über Cystein (gegebenenfalls mittels Disulfid-Verbrückung durch Cystein) miteinander oder mit anderen Elementen der Standards wie Aminosäuren, Spacer und/oder funktionelle Gruppen und/oder andere oben beschriebenen Elementen verknüpft, insbesondere kovalent gebunden. Ebenso sind in einer weiteren Variante die Epitope, und ein daran gebundener Spacer am Spacer nicht über eine Bindung zu einem Schwefelatom, nicht über eine Thioether-Bindung und/oder nicht über Cystein miteinander oder mit anderen Elementen der Standards wie Aminosäuren, weitere Spacer und/oder funktionelle Gruppen und/oder andere oben beschriebenen Elementen verknüpft, insbesondere kovalent gebunden.

In einer anderen Ausführungsform betrifft die vorliegende Erfindung ein Kit zur Verwendung in einem erfindungsgemäßen Verfahren, enthaltend:
- erfindungsgemäßen Standard,
- Sonde,
- erfindungsgemäße Fängermoleküle, Substrat und Sonden,
- Lösungen,
- Puffer,
wobei die Krankheit ausgewählt wird aus der Gruppe bestehend aus von Tauopathien, Parkinson-Krankheit und andere Synucleinopathien, und/oder Alzheimer-Demenz

Die Verbindungen und/oder Komponenten des Kits der vorliegenden Erfindung können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotitterplatte, absorbiert sein.

Ferner kann das Kit Anweisungen für den Gebrauch des Kits für eine Beliebige der Ausführungsformen enthalten.

Alle Verfahrensschritte nach der Entnahme der Probe werden ex vivo (in vitro), also außerhalb des menschlichen oder tierischen Körpers durchgeführt. Aggregat-Typen körpereigener fehlgefalteter Proteine werden bei folgenden Krankheit gefunden und können als Biomarker oder Teil eines Biomarkers eingesetzt werden: Tab. A, Zeilen 1-15

**Tab A:**

| | **Biomarker oder Teil davon** | **Krankheit** |
|---|---|---|
| 1* | Serum-Amyloid-A-Protein-Aggregate | AA-Amyloidose |
| 2* | IgG-light-chain-Aggregate | AL-Amyloidose |
| 3* | AApoAI-Aggregate | AApoAI-Amyloidose |
| 4* | AApoAII-Aggregate | AApoAII-Amyloidose |
| 5* | ATTR-Aggregate | ATTR-Amyloidose |
| 6* | DISC1-Aggregate | Schizophrenie und andere DISC1opathien |
| 7* | FUS-Aggregate | Amyotrophe lateralsklerose, Frontotemporale lobärdegeneration und andere FUS-Proteinopathien |
| 8* | IAPP-Aggregate | Diabetes Mellitus Typ 2 |
| 9* | SOD1-Aggregate | Amyotrophe Lateralsklerose |
| 10 | α-Synuclein-Aggregate | Parkinson-Krankheit und andere Synucleinopathien |
| 11 | Tau-Aggregate | Tauopathien |
| 12* | TDP-43-Aggregate | Amyotrophe Lateralsklerose, Frontotemporale Lobärdegeneration, Chronische Traumatische Enephalopathie und andere TDP-43 Proteinopathien |
| 13* | Huntingtin-Aggregate | Huntington-Krankheit |
| 14* | Lysozym-Aggregate | Familiäre viszerale Amyloidose |
| 15* | A-beta-Aggregate | Alzheimer Demenz |

| | | |
|---|---|---|
| * nicht erfindungsgemäß | | |

In einer Ausführung der vorliegenden Erfindung ist ein Biomarker der Tabelle A auch als Krankheitsindikator für eine andere Krankheit der Tabelle A zu verwenden, als in derselben Zeile des Biomarkers in der Tabelle angegeben.

Beispielsweise können Tauaggregate auch als Biomarker für Alzheimer Demenz dienen oder wie in der Tabelle gezeigt DISC1-Aggregate, FUS-Aggregate, α-Synuclein-Aggregate, Tau-Aggregate oder TDP-43-Aggregate für mehrere Krankheiten.

Beispielsweise können auch Mischaggregate, zum Beispiel aus Tau und α-Synuclein, als Biomarker für z.B. Parkinson dienen. Auch alle anderen Mischaggregate können als Biomarker verwendet werden.

Somit ist nicht nur die Diagnose von Kombinationen von Krankheiten möglich, sondern aus diesen Ergebnissen lassen sich andere Krankheiten auch ausschließen. Dadurch wird eine genauere Diagnose ebenfalls ermöglicht.

Gegenstand der vorliegenden Erfindung ist ein Verfahren zur Bestimmung von Krankheiten die Aggregate fehlgefalteter Proteine aufweisen, umfassend folgende Schritte:
i) quantitativen Bestimmung ex vivo wie vorstehend beschrieben von Krankheitsindikatoren;
ii) Vergleich dieser Daten mit den Normwerten;
iii) Feststellen einer signifikant unterschiedlichen Menge Krankheitsindikatoren bei diesem Vergleich;
iv) Zuordnung der Abweichung zu einer Krankheit ausgewählt aus der Gruppe bestehend aus Tauopathien, Parkinson-Krankheit und andere Synucleinopathien und/ oder Alzheimer'sche Demenz.

Auf dem eingesetzten Substrat können in mindestens zwei genau definierten Bereichen spezifische Fängermoleküle immobilisiert werden, die an unterschiedliche Aggregate körpereigene Proteine binden. In einer Alternative werden 3, 4, 5, 6, 7, 8, 9, 10, 11, 12, 13, 14, 15, 16 oder mehrere unterschiedliche spezifische Fängermoleküle auf genau definierten Bereichen des Substrats immobilisiert. Entsprechend der Bindung und anschließender Detektion der Aggregate ist dann eine Differenzialdiagnose möglich.

In einer Alternative können pro Well 16 oder mehrere unterschiedliche Fängermoluküle auf definierte Subflächen aufgebracht (gespottet) werden. Somit können verschiedene Aggregattypen und sogar heterogene Aggregate aus verschiedenen Monomeren in einer einzigen Probe identifiziert werden. Dadurch reichen kleine Mengen Körperflüssigkeiten aus. Außerdem kann so schon die Rasterposition zur Identifizierung der Aggregate dienen.

In einer Alternative kann ein Gemisch an verschiedenen Fängerantikörpern in einem einzigen Substrat (Well) immobilisiert werden, oder durch ein "Spotting"-Verfahren auf verschiedenen Positionen innerhalb einer einzigen Oberfläche, z.B. am Boden eines Mikrotiterplatten-Wells unterschiedliche Fängermoleküle (z.B. Antikörper) aufgebracht werden, die zum Beispiel spezifisch eine oder mehrere der Sonden binden oder die spezifisch eine oder mehrere der aggregierten Proteine binden. Durch den Einsatz verschiedener Sonden, die jeweils einen anderen Fluorophor tragen, können so verschiedene Aggregatspezies nahezu simultan in einem einzigen Probenaliquot analysiert werden.

Auch können so Aggregat-Mischformen, bestehend aus verschiedenen Protein-Monomeren identifiziert werden.

Somit ist nicht nur die Diagnose von Kombinationen von Krankheiten möglich, sondern aus diesen Ergebnissen lassen sich andere Krankheiten auch ausschließen. Dadurch wird eine genauere Diagnose ebenfalls ermöglicht.

Ein Vorteil des erfindungsgemäßen Verfahrens ist die Möglichkeit, mittels einer einzigen Patientenprobe, die eine Vielzahl von Entitäten umfasst, die mit Proteinaggregaten einhergehen, auf alle möglichen Aggregat-Typen zu untersuchen, die auf körpereigenen fehlgefalteten Proteinen beruhen. Weiterer Vorteil ist die Möglichkeit, die Proteinaggregate unmittelbar, ex vivo in Proben, wie Blut und Cerebrospinalflüssigkeitsproben oder Urin, Speichel, Schleimhaut, Biopsie-Material, insbesondere Blut und Cerebrospinalflüssigkeitsprobenunmittelbar auf Aggregat-Typen zu untersuchen.

Durch das erfindungsgemäße Verfahren können neue Krankheitsindikatoren qualitativ und/oder quantitativ bestimmt werden. Gegenstand der Erfindung sind somit auch Krankheitsindikatoren, Biomarker, gekennzeichnet durch die Anwesenheit eines Aggregat-Typs körpereigener fehlgefalteter Proteine, die erfindungsgemäß untersucht werden und ggf. die An- und/oder Abwesenheit mindestens eines weiteren -Aggregat-Typs.

Durch das erfindungsgemäße Verfahren und die neuen Biomarker kann eine der Krankheiten basierend auf fehlgefalteten Protein-Aggregaten (wie oben genannt ) identifiziert und von den anderen Krankheiten der Gruppe, ggf. mit ähnlichen oder übereinstimmenden Symptomen, eindeutig abgegrenzt werden.

### Beispiele

Erfindungsgemäß ist lediglich die Untersuchung von Tau und/oder α-Synuclein. Beispiele, die nicht die Untersuchung von Tau und/oder α-Synuclein betreffen, sind nicht erfindungsgemäß und dienen lediglich der Veranschaulichung.

### 1. Material und Methode

Als Probenträger wurden im Assay Multiwellplatten mit einem 170 µm starken Glasboden eingesetzt (SensoPlate Plus 384 Greiner Bio One, Kremsmünster, Österreich). Alle zum Einsatz kommenden Reagenzien und Lösungen wurden im höchsten Reinheitsgrad bezogen und vor Verwendung partikelfrei sterilisiert.

Im ersten Schritt wurde jede Probenkammer (Well) mit 100 µl Natronlauge (5 M) gefüllt, 15 Minuten bei Raumtemperatur inkubiert, dreimal mit Wasser gespült, mit 100 µl Salzsäure versetzt und erneut 15 Minuten bei Raumtemperatur inkubiert. Nach dreimaligem Waschen mit Wasser und zweimaligem Waschen mit Ethanol wurden die Wells unter Stickstoff-Atmosphäre getrocknet.

Zur Erzeugung von Aminogruppen auf der Glasoberfläche wurden jeweils 20 µl Ethanolamin (5,6 M) in die Wells gegeben und über Nacht bei Raumtemperatur inkubiert. Die Wells wurden dreimal mit DMSO, zweimal mit Ethanol gespült und unter Stickstoff-Atmosphäre getrocknet.

Heterobiofunktionales Polyethylenglykol (NHS-PEG-COOH, MW 3.400 Da) wurde zu 50 mM in DMSO bei 70 °C für 1 min gelöst, auf Raumtemperatur abgekühlt und auf 2% Triethylamin eingestellt. Von dieser Lösung wurde jeweils 15 µl in die Wells gegeben und für mindestens eine Stunde bei Raumtemperatur inkubiert. Die Lösung wurde aus den Wells entfernt, und die Wells werden dreimal mit Wasser gewaschen.

Zur Aktivierung der PEG-Beschichtung wurden NHS und EDC (Carbodiimid) zu jeweils 100 mM in MES-Puffer (0,1 M, pH 5, MES: 2-N-Morpholinoethansulfonsäure) gelöst und im Verhältnis 1:1 zu Endkonzentrationen von jeweils 50 mM gemischt. Je 30 µl dieser Lösung wurde in die Wells gefüllt und 30-60 Minuten inkubiert. Nach Entfernen der Lösung wurden die Wells dreimal mit MES-Puffer (0,1 M, pH 5) gewaschen.

Der Fänger-Antikörper wurde auf 30 ng/µl in PBS verdünnt. Davon wurden 15 µl in die Wells gegeben und für 1-3 Stunden bei Raumtemperatur inkubiert. Anschließend wurden die Lösung entfernt und dreimal mit PBST (PBS mit Tween 20), dann dreimal mit PBS gewaschen.

3% BSA wurde zuvor bei 100.000 g (1 Stunde bei 4°C) zentrifugiert. Je 50 µl des Überstands wurde in die Wells gefüllt und für eine Stunde bei Raumtemperatur inkubiert. Die BSA-Lösung wurde entfernt und dreimal mit PBST gewaschen.

Die Probe (z.B. Aggregate aus rekombinantem Protein und natürliche Patientenprobe) wurde - sofern erforderlich - in PBS verdünnt und davon jeweils 15 µl in die Wells gegeben. Die Multiwellplatte wurde bei 1.000g für eine Stunde bei 4 °C in einem Ausschwingrotor zentrifugiert. Der Überstand wurde entfernt und die Wells dreimal mit PBST und dreimal mit PBS gewaschen.

Als Detektionssonden wurden fluoreszenzmarkierte Antikörper eingesetzt. Diese werden auf 1-2 ng/µl in PBS verdünnt und mit 1.5% BSA versetzt. Die Ansätze wurden bei 100.000 g für eine Stunde bei 4 °C zentrifugiert. Je 15 µl des Überstands wurde in die Wells gefüllt und für 1-2 Stunden bei Raumtemperatur inkubiert. Anschließend wurde die Lösung entfernt und fünfmal mit PBST und fünfmal mit PBS gewaschen.

Die Oberflächenfluoreszenz wurde mittels Total Internal Reflection Fluoreszenz Mikroskopie (TIRFM) visualisiert. Alternativ können die Proteinpartikel mit konfokalem Laser Scanning auf Ebene der Glasoberfläche sichtbar gemacht werden. Pro Well wurden bis zu 50 Einzelbilder (1000x1000 Pixel, 114 nm/pixel) pro Fluoreszenzkanal mit einer hochsensitiven CCD-Kamera abgebildet.

Hintergrundsignale in den Bilddaten wurden über einen Intensitätsschwellenwert bereinigt. Die mittlere Anzahl der Pixel mit Graustufenwerten oberhalb des Schwellenwerts wurde bestimmt (sFIDA-readout). Kamen mehrere Detektionssonden zum Einsatz, wurden ausschließlich die in allen Fluoreszenzkanälen colokalisierten Ereignisse gewertet.

### 2. sFIDA Protokoll

### 2.1. Vorbehandlung

- 15 min NaOH (5M) 100 µl/Well
- 3x H₂O
- 15 min HCl (1M) 100 µl/Well
- 3x H₂O
- 2x EtOH spülen
- Mit N₂ trocknen

### 2.2. Glasaktivierung (Aminogruppen)

| | |
|---|---|
| • | 20 µl/Well Ethanolamin (5,6 M) 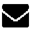 *Inkubation* ÜN (über Nacht) Raumtemperatur oder länger 4°C |
| • | 3x DMSO |
| • | 2x EtOH |
| • | Mit N₂ trocknen |

### 2.3. Spacer NHS-PEG-COOH

- 17 mg PEG in 100 µl DMSO bei 70°C (kurz) lösen, abkühlen lassen, + 2 µl Triethylamin (TEA)
- 15 µl/Well 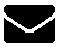 *Inkubation*: mind. 1 h
- 3× H₂O

### 2.4. PEG Aktivierung mit NHS/EDC (50mM)

- 5,8 mg NHS und 9,6 mg EDC in 500 µl MES (0,1M) verdünnen, direkt vor dem auftragen 1:1 mischen
- 30 µl/Well 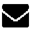 *Inkubation*: 30 min - max. 1 h
- zügig 3x MES (0,1M)

### 2.5. Capture

- Capture-Antikörper in PBS verdünnen (30ng/µl)
- 15 µl/Well -> Inkubation 1-3 h RT oder länger 4°C
- 3× PBST, 3x PBS

### 2.6. Blocken

| | |
|---|---|
| • | 3% BSA 1 h bei 100.000 x g, 4°C (Rotor TLA-45) |
| • | 50 µl/Well 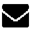 *Inkubation* 1 h |
| • | 3x PBST |
| • | 3x PBS |
| | Tag 2 |

### 2.7. Target

- 15 µl Target, verdünnt in PBS
- *Inkubation* 1h zf, 1000g , 25°C,
- HH: 3x 0,2% SDS/PBS; 5x PBST; 5x PBS
- Plasma und rekombinante Aggregate: 3x PBST; 3x PBS

### 2.8. Detektionsantikörper

- 1-2 ng/µl in 1,5% BSA mit PBS nach 100.000 x g, 4°C (Rotor TLA-45)
- je 15 µl Überstand/Well 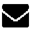 *Inkubation* >=1-2h, RT
- 5x PBST
- 5x PBS
(für Waschschritte wurden jeweils 100µl der entsprechenden Lösung verwendet)

### 3. Anwendung mit Aggregaten aus rekombinantem Protein

Zunächst wurde die prinzipielle Machbarkeit des sFIDA-Assays zum Nachweis verschiedener Aggregatspezies demonstriert. Exemplarisch wurden Aggregate von verschiedenen Proteinen (AapoAI, AL, SOD1, ALys, AA, TDP-43) präpariert, und entsprechende Verdünnungsreihen im sFIDA-Assay untersucht. Dabei wurde als Fänger- und Detektionsantikörper jeweils derselbe, gegen das jeweilige Protein gerichtete Antikörper verwendet. Die Detektionssonde wurde jeweils mit Alexafluor 488 markiert. Alle Aggregate können konzentrationsabhängig bis in den picomolaren Bereich mittels sFIDA nachgewiesen werden.

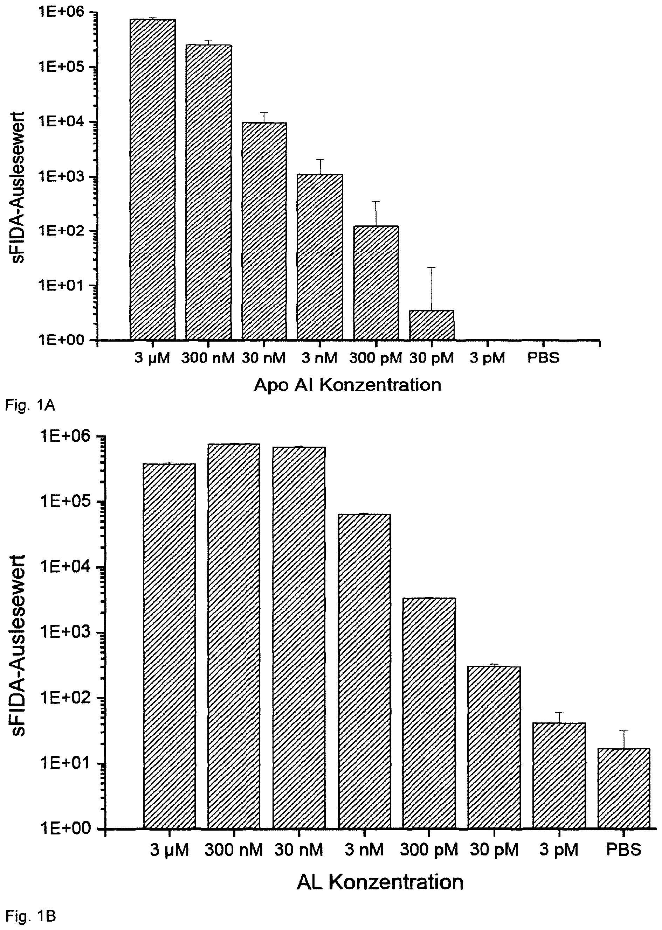

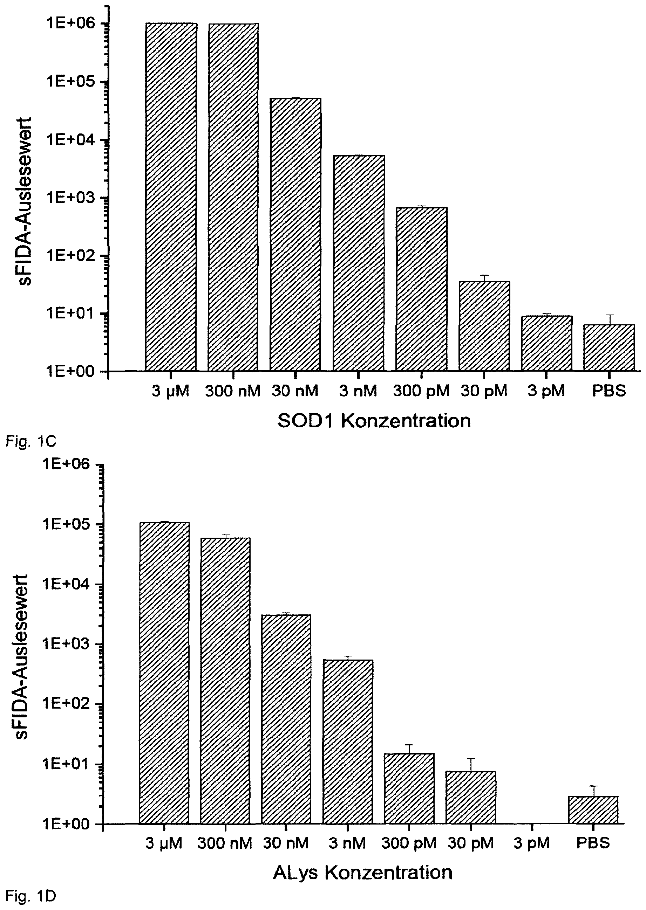

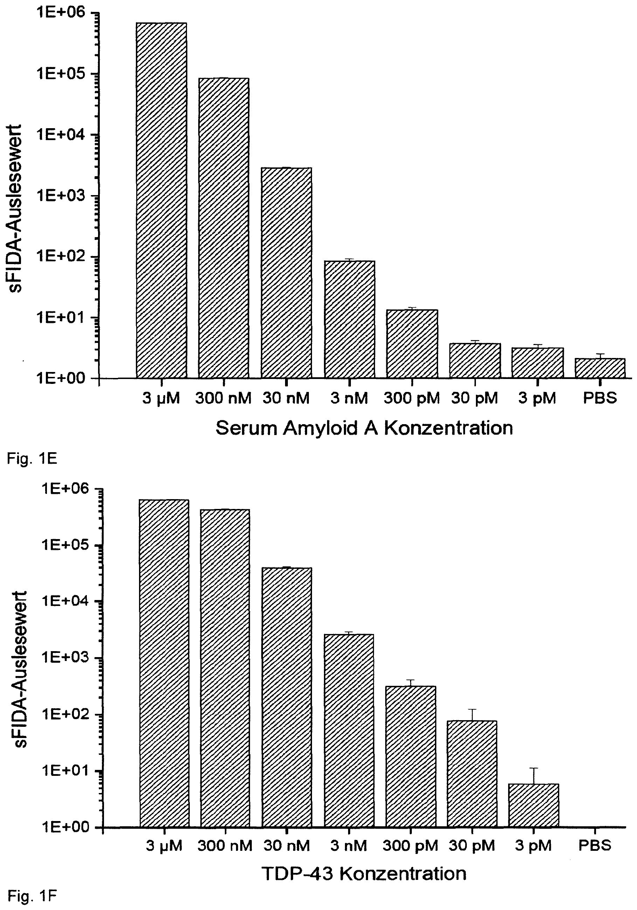

Fig. 1A-F: sFIDA von verschiedenen Proteinaggregaten. Die Proteinaggregate wurden in den angegebenen Konzentrationen in PBS-Puffer verdünnt und im sFIDA-Assay analysiert. Als Neagtivkontrolle wurde PBS-Puffer verwendet. **A)** ApoAI, Fänger- und Detektionssonde (AF488-markiert): EPR 1368Y **B)** AL, Fänger- und Detektionssonde (AF488-markiert): EPR 5367 **C)** SOD1, Fänger- und Detektionssonde (AF488-markiert): EPR 1726 **D)** ALys, Fänger- und Detektionssonde (AF488-markiert): EPR 2994 (2) **E**) AA, Fänger- und Detektionssonde (AF488-markiert): EPR 4134 **F**) TDP-43, Fänger- und Detektionssonde (AF488-markiert): EPR 5810

### 4. Anwendung an nativen Proben

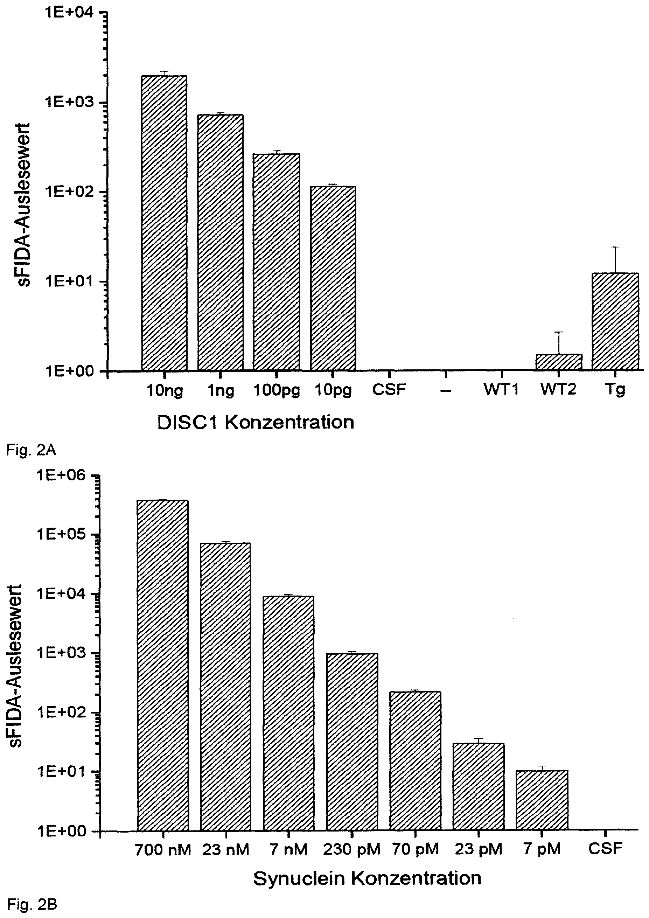

Um exemplarisch zu demonstrieren, dass Proteinaggregate in Blut- und Cerebrospinalflüssigkeitsproben (CSF-Proben) oder Urin, Speichel, Schleimhaut, Biopsie-Material detektiert werden können, wurden Proteinaggregate (α-Synuclein und DISC1) in CSF verdünnt und mittels sFIDA analysiert. Für den Nachweis von α-Synuclein wurde als Fänger-Antikörper Anti-aSyn-AB 2B2A11 und als Detektionssonde die fluoreszenzmarkierten Anti-aSyn-ABs 3H2897-AF633 bzw. 211-AF488 eingesetzt. Zur Detektion von DISC1-Aggregaten wurde Anti-DISC1-AK 14F2 als Fänger-Antikörper und 14F2-AF633 als Detektionssonde eingesetzt. Sowohl Synuclein als auch DISC1-Aggregate lassen sich konzentrationsabhängig bis in den picomolaren Bereich mittels sFIDA nachweisen (Siehe Fig. 2A und B).

Zur Untersuchung der molekularen Grundlagen der Schizophrenie wurde im Labor von Carsten Korth ein Rattenmodell entwickelt, welches das Protein DISC1 verstärkt exprimiert. Um zu überprüfen, ob dieses Protein nativ in Aggregaten vorliegt, wurden CSF-Proben eines transgenen Tiers mittels sFIDA analysiert. Im Vergleich zu zwei Kontrolltieren zeigte sich in der transgenen Probe ein deutlich erhöhter Titer an DISC1-Aggregaten (Fig. 2A, Proben WT1, WT2, Tg).

Fig. 2: sFIDA-Nachweis von Proteinaggregaten in CSF. Aggregate aus rekombinantem Protein wurden in humanem CSF verdünnt und in einem sFIDA-Assay untersucht. A) DISC1-Nachweis. Als Fängerantikörper und Detetktionssonde (AF633-markiert) kam mAB 14F2 zum Einsatz. Zusätzlich zu rekombinanten DISC1-Aggregaten (10pg-10ng) wurden CSF-Proben von Ratten (WT1, WT2, Tg) einer sFIDA-Analyse unterzogen. B) Synuclein-Nachweis mittels 2D sFIDA. Als Fänger-Antikörper wurde Anti-aSyn-AB 2B2A11 und als Detektionssonde die fluoreszenzmarkierten Anti-aSyn-ABs 3H2897-AF633 bzw. 211-AF488 eingesetzt. Nur die in beiden Farbkanälen kolokalisierten Signale oberhalb eines Schwellenwertes wurden berücksichtigt.

### 5. Differenzialdiagnostische Anwendung von sFIDA

Eine Probe wurde auf verschiedene Reaktionskammern (Wells) verteilt, welche mit den verschiedenen Fängerantikörpern beschichtet sind. Alternativ können ein Gemisch an verschiedenen Fängerantikörpern in einem einzigen Well immobilisiert werden, oder durch ein "Spotting"-Verfahren auf verschiedenen Positionen innerhalb einer einzigen Oberfläche, Z.B. am Boden eines Mikrotiterplatten-Wells unterschiedliche Fängerantikörper aufgebracht werden.

Durch den Einsatz verschiedener Sonden, die jeweils einen anderen Fluorophor tragen, wurden so verschiedene Aggregatspezies nahezu simultan in einem einzigen Probenaliquot analysiert.

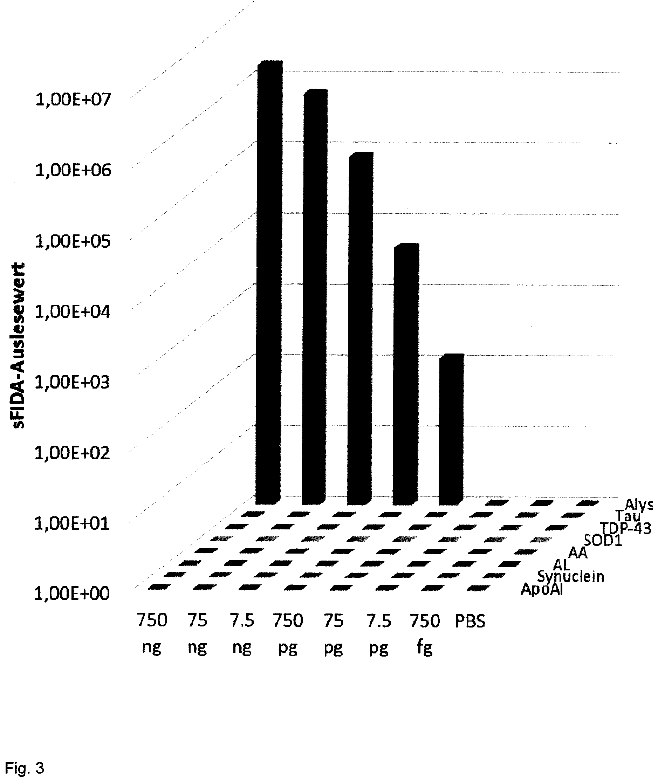

In diesem Ausführungsbeispiel wurde gezeigt, dass ein ALys-spezifischer sFIDA-Assay (Anti-Lysozym Antikörper als Sonde und Fänger) in einem breiten Konzentrationsbereich nicht mit anderen Proteinaggregaten kreuzreagiert (Abb. 3). Die Anti-Lysozym Antikörper als Sonde und Fänger interagieren nur mit Aggregaten des Typs ALys.

In einem weiteren Beispiel wurde gezeigt, dass die Anwesenheit andere Proteine keinen negativen Einfluss auf die Nachweisbarkeit von Amyloid beta-Aggregaten haben. Dazu wurde ein Gemisch aus Amyloid-beta-Aggregaten und Tau-Aggregaten einer sFIDA-Analyse unterzogen.

Fig. 3. Spezifitätsanalyse. In einem ALys-spezifischen sFIDA-Assay mit Fängerantikörper Rabbit monoclonal EPR2994(2) und Detektionssonde EPR2994(2)-AF488 wurden neben einer Verdünnung von rekombinanten ALys-Aggregaten andere Proteinaggregate (Tau, TDP-43, SOD1, AA, AL, Synuclein, ApoAl) auf Kreuzreaktivität geprüft.

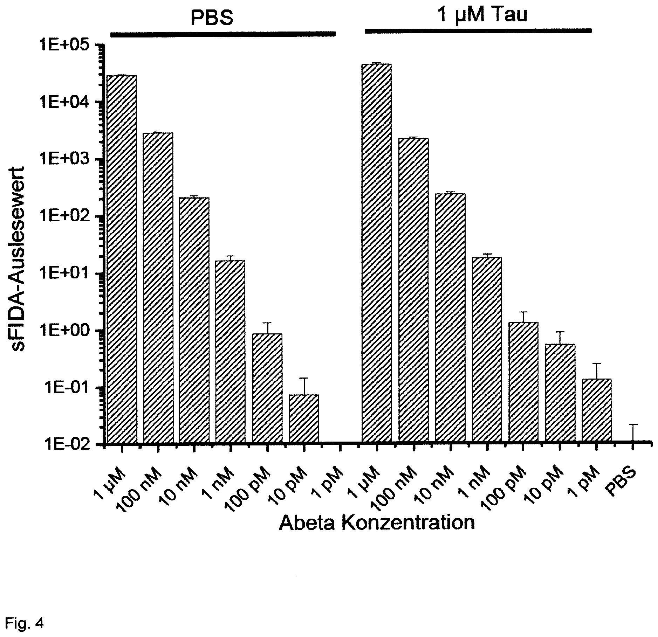

Fig. 4: Abeta-spezifischer sFIDA in Gegenwart von Tau-Protein. Proteinaggregate aus Abeta wurden in den angegebenen Konzentrationen verdünnt und in An- und Abwesenheit von 1 µM Tau-Protein-Aggregat in einem Abeta-spezifischen sFIDA-Assay (Detektionssonde 6E10/ Atto488, Capture Nab228) analysiert.

## Patentansprüche

1. Verfahren zur qualitativen und/oder quantitativen Bestimmung von Krankheitsindikatoren, **dadurch gekennzeichnet, dass** eine Probe auf mindestens zwei Aggregat-Typen körpereigener fehlgefalteter Proteine untersucht wird, wobei die mindestens zwei Aggregat-Typen körpereigener fehlgefalteter Proteine ausgewählt werden aus der Gruppe bestehend aus Tau-Aggregaten und a-Synuclein-Aggregaten und Mischaggregaten aus Tau und α-Synuclein, umfassend folgende Schritte:
vor Schritt a) Immobilisierung von Fängermolekülen auf einem Substrat, wobei die Fängermoleküle spezifische Anti-Körper gegen ein Epitop der Proteine aufweisen, die die Aggregate bilden,
a) Auftragen der zu untersuchenden Probe auf das Substrat, wobei als zu untersuchende Probe körpereigene Flüssigkeiten oder Gewebe ex vivo eingesetzt werden und wobei die Probe ohne weitere Aufarbeitung und/oder Behandlung auf mindestens zwei unterschiedliche Aggregat-Typen körpereigener fehlgefalteter Proteine untersucht wird,
b) Hinzufügen von für die Detektion gekennzeichneten Sonden, die durch spezifische Bindung an das jeweilige Aggregat dieses markieren, wobei als Sonden Antikörper eingesetzt werden, wobei mindestens 2 unterschiedliche Sonden eingesetzt werden, die sich sowohl bezüglich ihrer spezifischen Bindung an die Aggregate als auch bezüglich ihrer unterschiedlichen Kennzeichnung unterscheiden
und
c) Detektion der markierten Aggregate, wobei die Detektion in Schritt c) mittels einem Verfahren mit ortsaufgelöstem Signal erfolgt,
wobei
- Schritt b) vor Schritt a) durchgeführt werden kann,
und
- die Krankheit ausgewählt ist aus der Gruppe bestehend aus:
Tauopathien, Parkinson-Krankheit und andere Synucleinopathien und/oder Alzheimer'sche Demenz,
wobei eine Detektion von Monomeren ausgeschlossen wird, indem eine der Detektionssonden identisch mit dem Fängermolekül ist oder beide ein überlappendes Epitop erkennen,
und/oder indem Signale mit einer niedrigen Intensität durch ein Intensität-cut-off nicht gewertet werden,
und/oder durch Kreuzkorrelation.

2. Verfahren nach Anspruch 1, **dadurch gekennzeichnet, dass** die Probe auf mindestens zwei unterschiedliche Aggregat-Typen in einem Verfahrensschritt untersucht wird.

3. Verfahren nach Anspruch 1, **dadurch gekennzeichnet, dass** die Probe auf mindestens zwei unterschiedliche Aggregat-Typen auf demselben Substrat untersucht wird.

4. Verfahren nach Anspruch 1, **dadurch gekennzeichnet, dass** die Detektion in Schritt c) mittels sFIDA-Verfahren erfolgt.

5. Verfahren nach einem der vorangehenden Ansprüche, **dadurch gekennzeichnet, dass** die Fängermoleküle und/oder die Sonde mit Fluoreszenzfarbstoffen gekennzeichnet sind.

6. Verfahren nach einem der vorangehenden Ansprüchen, **dadurch gekennzeichnet, dass** die Fängermoleküle und/oder die Sonden spezifische Anti-Körper gegen ein Epitop der Proteine aufweisen, die die Aggregate bilden.

7. Verfahren nach Anspruch 1, **dadurch gekennzeichnet, dass** ein interner oder externer Standard eingesetzt wird.

8. Verwendung eines Standards zur selektiven Quantifizierung und/oder Charakterisierung der Krankheitsindikatoren in einem Verfahren gemäß Anspruch 1, **dadurch gekennzeichnet, dass** es sich bei dem Standard um ein Dendrimer handelt, welches ein zentrales Gerüstmolekül und Epitope der Proteine α-Synuclein-Aggregate oder Tau-Aggregate gemäß SEQ ID NO: 10 oder SEQ ID NO: 11 enthält, wobei sowohl Standards, die aus den gleichen Monomer-Sequenzen aufgebaut sind, als auch solche, die aus unterschiedlichen Monomer-Sequenzen aufgebaut sind verwendet werden.

9. Verfahren zur Bestimmung von Krankheiten die Aggregate fehlgefalteter Proteine aufweisen, umfassend folgende Schritte:
i) quantitativen Bestimmung ex vivo nach Anspruch 1 von Krankheitsindikatoren;
ii) Vergleich dieser Daten mit den Normwerten;
iii) Feststellen einer signifikant unterschiedlichen Menge Krankheitsindikatoren bei diesem Vergleich;
iv) Zuordnung der Abweichung zu einer Krankheit ausgewählt aus der Gruppe bestehend aus Tauopathien, Parkinson-Krankheit und andere Synucleinopathien und/oder Alzheimer'sche Demenz.

10. KIT zur Verwendung in einem Verfahren nach Anspruch 9 enthaltend:
- Standard nach Anspruch 8,
- Sonde,
- Fängermoleküle, Substrat und Sonden nach Anspruch 1,
- Lösungen,
- Puffer,
wobei die Krankheit ausgewählt wird aus der Gruppe bestehend aus von Tauopathien, Parkinson-Krankheit und andere Synucleinopathien, und/oder Alzheimer-Demenz.

## Claims

1. Method for the qualitative and/or quantitative determination of disease indicators, **characterized in that** a sample is analysed for at least two aggregate types of endogenous misfolded proteins, wherein the at least two aggregate types of endogenous misfolded proteins are selected from the group consisting of tau aggregates and α-synuclein aggregates and mixed aggregates of tau and α-synuclein, comprising the following steps:
before step a) immobilisation of capture molecules on a substrate, wherein said capture molecules have specific anti-bodies against an epitope of the proteins forming the aggregates,
a) applying the sample to be analysed to the substrate, wherein body fluids or tissues are used ex vivo as the sample to be analysed and wherein the sample is analysed without further processing and/or treatment for at least two different aggregate types of endogenous misfolded proteins,
b) adding probes labelled for the detection which label the respective aggregate by specific binding to it, wherein antibodies are used as probes, wherein at least 2 different probes are used which differ both with respect to their specific binding to the aggregates as well as with respect to their different labeling
and
c) detection of the labelled aggregates, wherein the detection in step c) is carried out by means of a method with spatially resolved signal,
wherein
- step b) can be carried out before step a),
and
- the disease is selected from the group consisting of:
tauopathies, Parkinson's disease and other synucleinopathies and/or Alzheimer's dementia,
wherein a detection of monomers is excluded by one of the detection probes being identical to the capture molecule or both detecting an overlapping epitope,
and/or by not evaluating signals with a low intensity by an intensity cut-off, and/or by cross-correlation.

2. Method according to claim 1, **characterized in that** the sample is analysed for at least two different aggregate types in one method step.

3. Method according to claim 1, **characterized in that** the sample is analysed for at least two different aggregate types on the same substrate.

4. Method according to claim 1, **characterized in that** the detection in step c) is carried out by means of sFIDA method.

5. Method according to any one of the preceding claims, **characterized in that** the capture molecules and/or the probe are labelled with fluorescent dyes.

6. Method according to any one of the preceding claims, **characterized in that** the capture molecules and/or the probes have specific anti-bodies against an epitope of the proteins forming the aggregates.

7. Method according to claim 1, **characterized in that** an internal or external standard is used.

8. Use of a standard for selective quantification and/or characterisation of the disease indicators in a method according to claim 1, **characterised in that** the standard is a dendrimer which contains a central scaffold molecule and epitopes of the proteins a-synuclein aggregates or tau aggregates according to SEQ ID NO: 10 or SEQ ID NO: 11, wherein both standards build-up from the same monomer sequences as well as those build-up from different monomer sequences are used.

9. Method for the determination of diseases having aggregates of misfolded proteins, comprising the following steps:
i) quantitative determination ex vivo according to claim 1 of disease indicators;
ii) comparing these data with standard values;
iii) detecting a significantly different set of disease indicators in this comparison;
iv) assigning the difference to a disease selected from the group consisting of tauopathies, Parkinson's disease and other synucleinopathies and/or Alzheimer's dementia.

10. KIT for use in a method according to claim 9 comprising:
- standard according to claim 8,
- probe,
- capture molecules, substrate and probes according to claim 1,
- solutions,
- buffer,
wherein the disease is selected from the group consisting of tauopathies, Parkinson's disease and other synucleinopathies, and/or Alzheimer's dementia.

## Revendications

1. Procédé de détermination qualitative et/ou quantitative d'indicateurs de maladie,
**caractérisé en ce qu'**un échantillon est analysé quant à au moins deux types d'agrégats de protéines endogènes mal repliées, lesdits au moins deux types d'agrégats de protéines endogènes mal repliées étant choisis dans le groupe constitué par les agrégats de tau et les agrégats d'α-synucléine et les agrégats mixtes de tau et d'a-synucléine, comprenant les étapes suivantes consistant à :
avant l'étape a), immobiliser des molécules de capture sur un substrat, les molécules de capture présentant des anticorps spécifiques contre un épitope des protéines constituant les agrégats,
a) appliquer l'échantillon à analyser sur le substrat, en utilisant comme échantillon à analyser des liquides ou des tissus endogènes ex vivo et en analysant l'échantillon sans autre traitement et/ou manipulation quant à au moins deux types différents d'agrégats de protéines endogènes mal repliées,
b) ajouter des sondes marquées pour la détection, qui marquent l'agrégat respectif par une liaison spécifique à celui-ci, des anticorps étant utilisés comme sondes, au moins 2 sondes différentes étant utilisées, qui se distinguent aussi bien par leur liaison spécifique aux agrégats que par leur marquage différent,
et
c) détecter les agrégats marqués, la détection dans l'étape c) s'effectuant par une méthode avec signal à résolution spatiale,
où
- l'étape b) peut être effectuée avant l'étape a),
et
- la maladie est choisie dans le groupe constitué par :
tauopathies, maladie de Parkinson et autres synucléinopathies et/ou démence d'Alzheimer,
la détection de monomères est exclue du fait que l'une des sondes de détection est identique à la molécule de capture ou que toutes les deux reconnaissent un épitope chevauchant,
et/ou du fait que les signaux de faible intensité ne sont pas évalués par le biais d'une coupure d'intensité (cut-off),
et/ou par corrélation croisée.

2. Procédé selon la revendication 1,
**caractérisé en ce que** l'échantillon est analysé quant à au moins deux types d'agrégats différents en une seule étape du procédé.

3. Procédé selon la revendication 1,
**caractérisé en ce que** l'échantillon est analysé quant à au moins deux types d'agrégats différents sur le même substrat.

4. Procédé selon la revendication 1,
**caractérisé en ce que** la détection à l'étape c) est effectuée au moyen d'une méthode sFIDA.

5. Procédé selon l'une des revendications précédentes,
**caractérisé en ce que** les molécules de capture et/ou la sonde sont marquées par des colorants fluorescents.

6. Procédé selon l'une des revendications précédentes,
**caractérisé en ce que** les molécules de capture et/ou les sondes présentent des anticorps spécifiques contre un épitope des protéines constituant les agrégats.

7. Procédé selon la revendication 1,
**caractérisé en ce que** l'on utilise un étalon interne ou externe.

8. Utilisation d'un étalon pour la quantification et/ou caractérisation sélective des indicateurs de maladie dans un procédé selon la revendication 1, **caractérisée en ce que** l'étalon est un dendrimère qui contient une molécule squelette centrale et des épitopes des protéines d'agrégats d'a-synucléine ou d'agrégats de tau selon SEQ ID NO : 10 ou SEQ ID NO : 11,
en utilisant aussi bien des étalons constitués des mêmes séquences de monomères que des étalons constitués de séquences de monomères différentes.

9. Procédé de détermination de maladies présentant des agrégats de protéines mal repliées, comprenant les étapes suivantes :
i) détermination quantitative ex vivo d'indicateurs de maladies selon la revendication 1 ;
ii) comparaison de ces données aux valeurs normales ;
iii) détection d'une quantité significativement différente d'indicateurs de maladie lors de cette comparaison ;
iv) attribution de l'écart différence à une maladie choisie dans le groupe constitué par les tauopathies, la maladie de Parkinson et autres synucléinopathies, et/ou la démence d'Alzheimer.

10. Kit destiné à être utilisé dans un procédé selon la revendication 9, comprenant :
- l'étalon selon la revendication 8,
- une sonde,
- des molécules de capture, le substrat et les sondes selon la revendication 1,
- des solutions,
- un tampon,
la maladie étant choisie dans le groupe constitué par les tauopathies, la maladie de Parkinson et autres synucléinopathies, et/ou la démence d'Alzheimer.
